# EUROPEAN PATENT APPLICATION

(11) **EP 2 923 764 A1**
(43) Date of publication of application: **30.09.2015**
(21) Application number: 15161047.4
(22) Date of filing: 26.03.2015
(51) Int. Cl.: B01L 3/00

(54) **CARTRIDGE FOR NUCLEIC ACID AMPLIFICATION REACTION AND NUCLEIC ACID AMPLIFICATION DEVICE**

(30) Priority: 28.03.2014 JP 2014068233
(71) Applicant: Seiko Epson Corporation, Tokyo 163-0811 (JP)
(72) Inventor: Saito, Yuji, Nagano, 392-8502 (JP); Takagi, Fumio, Nagano, 392-8502 (JP)
(74) Representative: Hoffmann Eitle

(57) **Abstract**

A cartridge for a nucleic acid amplification reaction includes a tube which has a longitudinal direction and has, in the inside thereof, an elution solution plug formed of an elution solution, which undergoes phase separation from oil and causes nucleic acid to be eluted from particulates bound to the nucleic acid, and a plug of a first liquid formed of oil and a first additive, and a nucleic acid amplification reaction container which communicates with the tube. The nucleic acid amplification reaction container contains a second liquid formed of oil and a second additive, the first additive is selected from a group consisting of carbinol-modified silicone resin, carboxyl-modified silicone resin, amino-modified silicone resin, polyether-modified silicone resin, silanol-modified silicone resin, and fluoro-modified silicone resin, the second additive is selected from a group consisting of carbinol-modified silicone resin, carboxyl-modified silicone resin, amino-modified silicone resin, polyether-modified silicone resin, silanol-modified silicone resin, and fluoro-modified silicone resin, and the concentration of the first additive contained in the first liquid is higher than the concentration of the second additive contained in the second liquid.

## Description

### BACKGROUND

### 1. Technical Field

The present invention relates to a cartridge for a nucleic acid amplification reaction and a nucleic acid amplification device.

### 2. Related Art

In recent years, with the advances in techniques using genes, medical treatment using genes, such as gene diagnosis or gene therapy, has been attracting attention. In the field of agriculture and livestock, many techniques using genes have been developed for breed identification and breeding. As a technique for using genes, a nucleic acid amplification technique, such as a PCR (Polymerase Chain Reaction) method, has widely been used. Nowadays, the PCR method is an essential technique for revealing information of biological substances.

In the PCR method, a technique which performs a reaction using a container, called a tube or a chip (hereinafter, referred to as a biochip), for performing a biochemical reaction, is generally used. However, in the technique of the related art, a required amount of reagent or the like is great, a device becomes complicated so as to realize a thermal cycle necessary for a reaction, and a reaction takes a lot of time. For this reason, a biochip or a reaction device for performing PCR in a short time using an extremely small amount of reagent or specimen is required.

In order to solve such a problem, JP-A-2009-136250 discloses a biochip and a device which apply a thermal cycle by reciprocating a reaction solution contained in a state of a droplet in a tube filled with a liquid (mineral oil or the like), which is not mixed with the reaction solution and has specific gravity different from the reaction solution, and perform a reaction.

When the biochip disclosed in JP-A-2009-136250 is used for measuring fluorescence from the outside of the container to detect an amplified product, the container should be formed of a transparent material. Examples of the transparent material include resin and heat resistant-glass, but these materials are easily charged by friction or the like. If the inner surface of the container is subjected to hydrophilic treatment, charging can be suppressed. However, the reaction solution which is an aqueous solution is adhered to the container and the movement of the reaction solution is hindered. Accordingly, it is difficult to apply the hydrophilic treatment to the biochip.

As the liquid which is not mixed with the reaction solution and has specific gravity different from the reaction solution, in terms of stability to heat or the reaction solution, silicone oil or mineral oil can be used, but these oils are generally insulators. Accordingly, a droplet of the reaction solution introduced into oil is easily polarized. For this reason, if a container made of a transparent material is filled with oil and the reaction solution is introduced into the container, an electric field occurs between the reaction solution and the container, the reaction solution is attracted to the inner wall of the container and adhered to the container, or the reaction solution floats in oil by repulsion. In such a state, if PCR of a type (hereinafter, in this specification, this system is called "elevation type") described in JP-A-2009-136250, in which a reaction solution is moved in a biochip by gravity, is performed, the reaction solution is not appropriately moved, and a desired thermal cycle may not be performed.

JP-A-2012-125169 discloses a biochip which removes an electric charge occurring in the biochip, and when a container filled with a liquid, which is not mixed with a reaction solution, is used in order to subject a stable thermal cycle to the reaction solution, sets the volume intrinsic resistance of the liquid to be greater than 0 Ω·cm and equal to or less than 5X10¹³ Ω·cm.

Even if charging is prevented as described in JP-A-2012-125169, a droplet is still adhered to the container, the droplet cannot move in oil by gravity, and the addition of an additive to oil in a nucleic acid amplification reaction container is considered. Meanwhile, the activity of the nucleic acid amplification reaction reagent in the nucleic acid amplification reaction container is hindered according to an additive.

### SUMMARY

An advantage of some aspects of the invention is to provide a cartridge for a nucleic acid amplification reaction capable of preventing activity of a nucleic acid amplification reaction reagent in a nucleic acid amplification reaction container from being hindered.

An aspect of the invention is directed to a cartridge for a nucleic acid amplification reaction including a tube which has a longitudinal direction and has, in the inside thereof, an elution solution plug formed of an elution solution, which undergoes phase separation from oil and causes nucleic acid to be eluted from particulates bound to the nucleic acid, and a plug of a first liquid formed of oil and a first additive, and a nucleic acid amplification reaction container which communicates with the tube. The nucleic acid amplification reaction container contains a second liquid formed of oil and a second additive, the first additive is selected from a group consisting of carbinol-modified silicone resin, carboxyl-modified silicone resin, amino-modified silicone resin, polyether-modified silicone resin, silanol-modified silicone resin, and fluoro-modified silicone resin, the second additive is selected from a group consisting of carbinol-modified silicone resin, carboxyl-modified silicone resin, amino-modified silicone resin, polyether-modified silicone resin, silanol-modified silicone resin, and fluoro-modified silicone resin, and the concentration of the first additive contained in the first liquid is higher than the concentration of the second additive contained in the second liquid. The additive may be one of X-22-160AS, X-22-3701E, KF-857, KF-859, KF-862, KF-867, KF-6017, KF-8005 (manufactured by Shin-Etsu Silicone Co., Ltd.), SR1000, SS4230, SS4267, YR3370, XS66-C1191, TSF4703, TSF4708, XF42-C5196, and XF42-C5197 (manufactured by Momentive Co., Ltd.). The first additive and the second additive may be the same silicone resin. The ratio of the volume, which is obtained by adding the volume of the second additive to the volume of the first additive, to the volume, which is obtained by adding the volume of the second liquid to the volume of the first liquid in the nucleic acid amplification reaction container when the plug of the first liquid is ejected to the nucleic acid amplification reaction container, may be equal to or greater than 1% (v/v) and equal to or less than 20% (v/v). The nucleic acid amplification reaction container contains a nucleic acid amplification reaction reagent. The nucleic acid amplification reaction reagent may be held in the second liquid. The nucleic acid amplification reaction reagent may be freeze-dried. The nucleic acid amplification reaction reagent may contain a surfactant, and the surfactant may be NP40, Triton-X100, or Tween20. The container may be made of polypropylene.

Another aspect of the invention is directed to a nucleic acid amplification device on which the cartridge for a nucleic acid amplification reaction described above is mounted.

### BRIEF DESCRIPTION OF THE DRAWINGS

The invention will be described with reference to the accompanying drawings, wherein like numbers reference like elements.
Figs. 1A and 1B are diagrams illustrating a cartridge.
Figs. 2A to 2C are diagrams illustrating the operation of the cartridge.
Figs. 3A to 3D are diagrams illustrating a tank.
Fig. 4 is a diagram illustrating a fixing claw, a guide plate, and a mounting portion.
Figs. 5A and 5B are diagrams illustrating the periphery of a PCR container.
Fig. 6A is a perspective view of the internal configuration of a PCR device. Fig. 6B is a side view of the main configuration of the PCR device.
Fig. 7 is a block diagram of the PCR device.
Fig. 8A is a diagram illustrating a rotary body. Fig. 8B is a diagram illustrating a state where the cartridge is mounted in the mounting portion of the rotary body.
Figs. 9A to 9D are diagrams illustrating a state of the PCR device when the cartridge is mounted.
Fig. 10 is a conceptual diagram of the behavior of magnetic beads when magnets are moved downward.
Figs. 11A to 11C are diagrams illustrating a nucleic acid elution process.
Fig. 12 is a conceptual diagram of the behavior of the magnetic beads when the magnets oscillate.
Fig. 13 is a table showing the presence/absence of oscillation of the magnets.
Figs. 14A to 14C are diagrams illustrating a droplet forming process.
Figs. 15A and 15B are diagrams illustrating a thermal cycle process.
Fig. 16 is a diagram showing a kit for nucleic acid extraction used in an example and a device after assembling.
Fig. 17 is a graph showing a result of real time PCR in an example.
Figs. 18A and 18B are graphs showing a result of comparison of preservation stability of a nucleic acid amplification solution in an example.
Fig. 19 is a graph showing a result of real time PCR in an example.

### DESCRIPTION OF EXEMPLARY EMBODIMENTS

Hereinafter, embodiments of the invention will be described in detail. The description of the specification makes those skilled in the art clearly see the objects, features, advantages, and ideas of the invention, and those skilled in the art can easily make the invention based on the description of the specification. The embodiments of the invention described below are preferred embodiments of the invention and are only for illustration and description, and the invention is not limited thereto. Those skilled in the art know for sure that the invention can be changed and modified in various ways based on the description of the specification within the intention and the scope of the invention described in the specification.

A cartridge for a nucleic acid amplification reaction according to an embodiment of the invention includes a tube which has a longitudinal direction and has, in the inside thereof, an elution solution plug, which undergoes phase separation from oil and causes nucleic acid to be eluted from particulates bound to the nucleic acid and a plug of a first liquid formed of oil and a first additive, and a nucleic acid amplification reaction container which communicates with the tube. The nucleic acid amplification reaction container contains a second liquid formed of oil and a second additive, the first additive is selected from a group consisting of carbinol-modified silicone resin, carboxyl-modified silicone resin, amino-modified silicone resin, polyether-modified silicone resin, silanol-modified silicone resin, and fluoro-modified silicone resin, the second additive is selected from a group consisting of carbinol-modified silicone resin, carboxyl-modified silicone resin, amino-modified silicone resin, polyether-modified silicone resin, silanol-modified silicone resin, and fluoro-modified silicone resin, and the concentration of the first additive contained in the first liquid is higher than the concentration of the second additive contained in the second liquid.

Hereinafter, the configuration of the cartridge for a nucleic acid amplification reaction will be described in detail. When describing a general configuration, the plug of the first liquid in the tube will be described as an oil plug. However, in the embodiment of the invention, one or more oil plugs in the tube become the plug of the first liquid.

### Cartridge

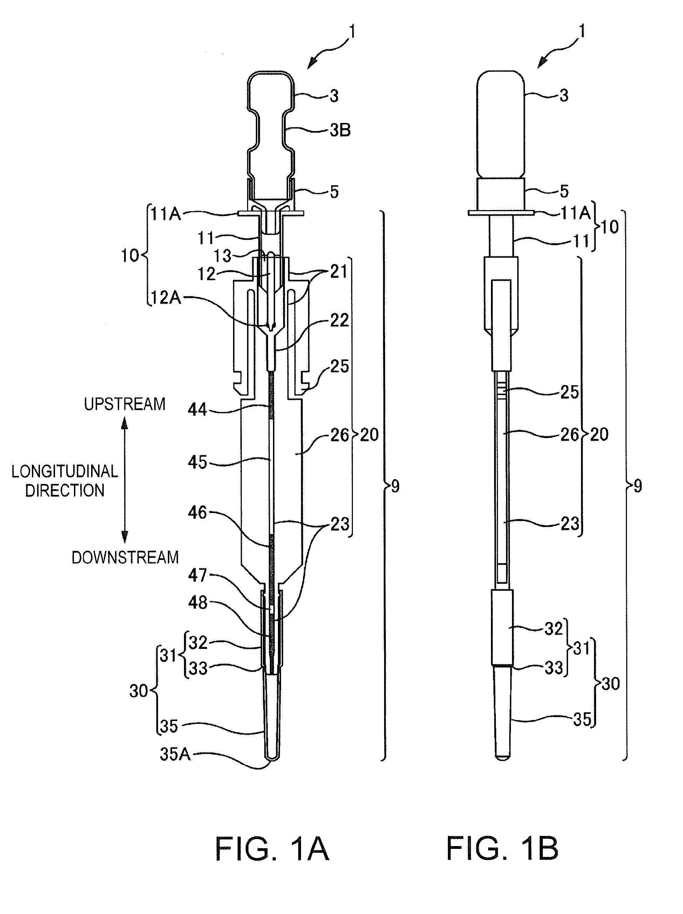

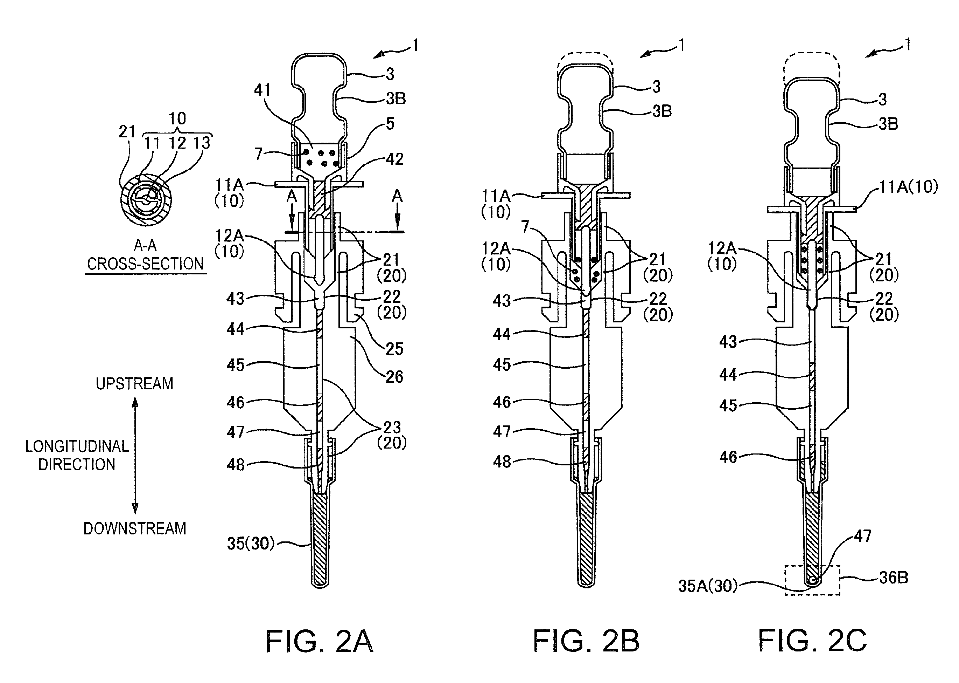

Figs. 1A and 1B are diagrams illustrating a cartridge 1. Figs. 2A to 2C are diagrams illustrating the operation of the cartridge 1. Fig. 2A is a diagram illustrating the initial state of the cartridge 1. Fig. 2B is a side view showing a state where a plunger 10 is pushed in the state of Fig. 2A such that a seal 12A comes into contact with a lower syringe 22. Fig. 2C is a diagram illustrating the cartridge 1 after the plunger 10 is pushed. First, a summary of a method of using the cartridge will be described.

The cartridge 1 is constituted by a tank 3 and a cartridge main body 9 including a PCR container 30. In a kit constituting the cartridge 1, an adapter 5 is prepared in advance together with the tank 3 and the cartridge main body 9. The tank 3 and the cartridge main body 9 are connected to each other through the adapter 5, whereby the cartridge 1 can be assembled. However, the tank 3 may be directly attached to the cartridge main body 9.

In the tank 3, a nucleic acid extraction process is performed, and nucleic acid is bound to magnetic beads 7. A tube 20 has a washing solution plug 45, an elution solution plug 47, and an oil plug. The magnetic beads 7 entered the tube 20 from the tank 3 move inside the tube 20 by moving magnets to the outside along the tube 20 and reach the elution solution plug 47 through the washing solution plug 45. The nucleic acid bound to the magnetic beads 7 is washed with the washing solution in the washing solution plug 45 and eluted in the elution solution plug 47.

In the PCR container 30 communicating with the tube 20, a thermal cycle process is performed. The PCR container 30 is filled with the second liquid. If pushed out from the tube 20 into the PCR container 30, the elution solution plug 47 becomes a droplet, and the elution solution 47 in the form of a droplet precipitates. The elution solution 47 precipitating into the PCR container 30 dissolves a freeze-dried nucleic acid amplification reaction reagent in the PCR container 30 and becomes a PCR solution. If a high temperature region 36A and a low temperature region 36B are formed in the PCR container 30 by an external heater, and the entire cartridge 1 is repeatedly moved upside down together with the heater, the PCR solution in the form of a droplet alternately moves between the high temperature region 36A and the low temperature region 36B, whereby a two-stage temperature process is performed and DNA is amplified.

In the following detailed description of the constituents of the cartridge 1, as shown in Fig. 2A, the direction along the long cartridge 1 is referred to as a "longitudinal direction", the tank 3 side is referred to as an "upstream side", and the PCR container 30 side is referred to as a "downstream side". The upstream side is simply referred to as an "upper side", and the downstream side is simply referred to as a "lower side".

### 1. Tank

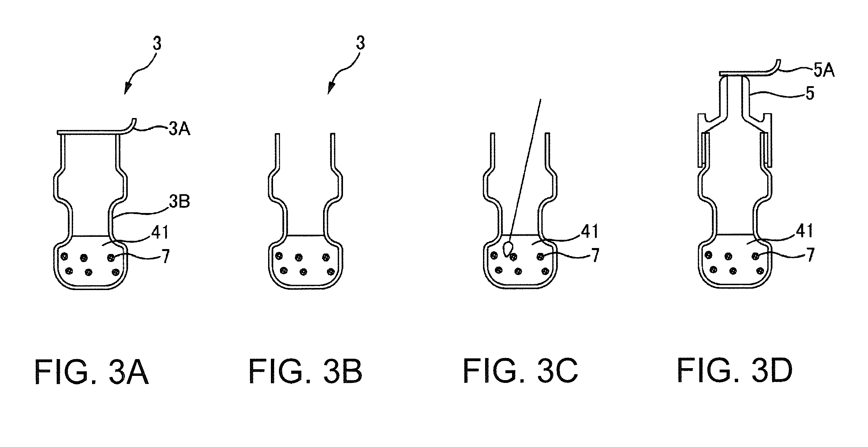

Figs. 3A to 3D are diagrams illustrating the tank 3.

The tank 3 prepared in advance in the kit contains a dissolution solution 41 and magnetic beads 7. A detachable lid 3A is attached to the opening of the tank 3 (see Fig. 3A) . As the dissolution solution 41, 5 M guanidine thiocyanate, 2% Triton X-100, and 50 mM Tris-HCl (pH 7.2) are used. An operator removes the lid 3A to open the opening of the tank 3 (see Fig. 3B), and dips a cotton swab smeared with a virus into the dissolution solution 41 in the tank 3 to collect the virus in the dissolution solution 41 (see Fig. 3C). When the liquid in the tank 3 is stirred, the tank 3 may be shaken in the state of Fig. 3C. In this case, however, the dissolution solution 41 easily overflows. Accordingly, as shown in Fig. 3D, it is preferable to shake the tank 3 after the adapter 5 covered with a lid 5A is attached to the opening of the tank 3. In this way, the substance in the tank 3 is stirred, and the virus particles are dissolved by the dissolution solution 41, whereby the nucleic acid is liberated, and silica coated on the magnetic beads 7 adsorbs the nucleic acid. The magnetic beads 7 correspond to the nucleic acid-binding solid-phase carriers. Thereafter, the operator detaches the lid 5A of the adapter 5 attached to the opening of the tank 3 and attaches the tank 3 to the cartridge main body 9 through the adapter 5 (see Fig. 2A).

The tank 3 is made of flexible resin and is dilatable. For example, examples of flexible resin include resin, such as polypropylene, polyethylene, and cycloolefin polymer (for example, ZEONEX (Registered Trademark) 480R). When the state of the plunger 10 slides and the state of the cartridge changes from the state of Fig. 2A to the state of Fig. 2B, the tank 3 dilates, and this prevents the pressure in the tube 20 from excessively increasing and prevents the liquid in the tube 20 from being pushed out to the downstream side. It is possible to form a deformation portion 3B in the tank 3 such that the tank 3 easily dilates.

A sample used for extracting and amplifying nucleic acid is not limited to viruses and may be cells. The sources of cells are not particularly limited and may be microorganisms, tissue fragments or blood of higher organisms, and the like.

The dissolution solution 41 refers to a liquid which causes the nucleic acid to be adsorbed onto particulates (for example, magnetic particles M) as the nucleic acid-binding solid-phase carriers. The dissolution solution 41 is not particularly limited as long as the solution contains a chaotropic substance, and may contain a surfactant to destroy the cell membrane or denature proteins contained in the cells. The surfactant is not particularly limited as long as the surfactant is generally used for extracting nucleic acid from cells and the like. Specific examples thereof include nonionic surfactants including triton-based surfactants, such as Triton-X or tween-based surfactants such as Tween-20, and anionic surfactants such as N-lauroylsarcosine sodium (SDS). It is particularly preferable to use nonionic surfactants at a concentration within a range of 0.1% to 2%. It is preferable for the dissolution solution to contain a reductant such as 2-mercaptoethanol or dithiothreitol. The dissolution solution 41 may be a buffer solution, and pH thereof is preferably neutral such as pH 6 to 8. Considering the above, specifically, it is preferable for the dissolution solution to contain 3 M to 7 M of a guanidine salt, 0% to 5% of a nonionic surfactant, 0 mM to 0.2 mM of EDTA, 0 M to 0.2 M of a reductant, and the like.

The chaotropic substance acts to generate chaotropic ions (monovalent anions having a large ion radius) in an aqueous solution and enhance water solubility of hydrophobic molecules. This substance is not particularly limited as long as the substance contributes to the adsorption of nucleic acid onto the solid-phase carriers. Specific examples thereof include guanidine thiocynate, guanidine hydrochloride, sodium iodide, potassium iodide, sodium perchlorate, and the like. Among these, guanidine thiocynate or guanidine hydrochloride having a potent protein denaturating action is preferable. The concentration of these chaotropic substances used varies with the type of each of the substances. For example, when guanidine thiocyanate is used, this substance is preferably used in a range of 3 M to 5.5 M, and when guanidine hydrochloride is used, this substance is preferably used at a concentration of 5 M or more.

It is preferable that the dissolution solution 41 contains alcohol or acetonitrile. In this case, the lower limit of the concentration of alcohol or the like is not particularly limited and is preferably equal to or greater than 10%, more preferably equal to or greater than 20%, still more preferably equal to or greater than 30%, and most preferably equal to or greater than 40%. The upper limit of the concentration of alcohol or the like is preferably equal to or less than 80%, more preferably equal to or less than 70%, still more preferably equal to or less than 60%, and most preferably equal to or less than 50%. The type of alcohol is not particularly limited, and examples thereof include methanol, ethanol, propanol, and the like. The addition of alcohol or the like to the dissolution solution can increase the effect of the adsorption of nucleic acid onto particles and the like and nucleic acid extraction efficiency when being observed as a device for nucleic acid extraction.

The tool for collecting the sample is not particularly limited, and instead of a cotton swab, a spatula, a rod, a scraper, or the like may be selected according to the purpose.

The internal volume of the tank 3 is not particularly limited, and may be, for example, from 0.1 mL to 100 mL. The material of the tank 3 is not particularly limited, and the tank 3 can be made of, for example, glass, resin, such as plastic, metal, or the like. Particularly, it is preferable to select transparent glass or resin as the material of the tank since the inside of the tank 3 can be observed from the outside. The tank 3 and each tube 20 may be formed by monolithic molding or may be detachable from each other. If a flexible material, such as rubber, elastomer, and polymer are used as the material of the tank 3, it is possible to increase the internal pressure of the tank 3 by deforming the tank 3 in a state where the tank 3 is covered with a lid. As a result, it is possible to push the content of the tube 20 out from the tip of the tube toward the outside from the inside of the tube.

### 2. Cartridge Main Body

The cartridge main body 9 has the plunger 10, the tube 20, and the PCR container 30.

### 2-1. Plunger

Hereinafter, the plunger 10 will be described referring to Figs. 2A to 2C.

The plunger 10 is a movable plunger which pushes out a liquid from the downstream side of the tube 20 functioning as a syringe. The plunger 10 functions to push out a predetermined amount of liquid in the tube 20 from the terminal of the tube 20 to the PCR container 30. The plunger 10 also functions to attach the tank 3 through the adapter 5.

The plunger 10 has a cylindrical portion 11 and a rod-like portion 12. The cylindrical portion 11 is provided on the tank 3 side (upstream side), and the rod-like portion 12 is provided on the tube 20 side (downstream side). The rod-like portion 12 is supported by two plate-like ribs 13 from the inner wall on the downstream side of the cylindrical portion 11. The downstream side of the rod-like portion 12 protrudes from the cylindrical portion 11 toward the downstream side.

The cylindrical portion 11 is opened toward the upstream side and the downstream side, and the inner wall of the cylindrical portion 11 becomes a path of a liquid. The adapter 5 fits to the opening on the upstream side (tank 3 side) of the cylindrical portion 11. In the plunger 10 of the cartridge main body 9 prepared in advance in the kit, a detachable lid may be attached to the opening on the upstream side of the cylindrical portion 11. The opening on the downstream side of the cylindrical portion 11 is positioned in the inside of an upper syringe 21 of the tube 20. The magnetic beads 7 introduced from the opening on the upstream side of the cylindrical portion 11 pass through the inside of the cylindrical portion 11, escape from the inside and outside of the ribs 13, come out from the opening on the downstream side of the cylindrical portion 11, and are introduced into the upper syringe 21 of the tube 20.

The downstream side of the cylindrical portion 11 fits to the inner wall of the upper syringe 21 of the tube 20. The cylindrical portion 11 can slide relative to the upper syringe 21 in the longitudinal direction while coming into contact with the inside of the upper syringe 21 of the tube 20.

In the periphery of the opening on the upstream side of the cylindrical portion 11, a mounting board 11A for mounting the adapter 5 is formed. The mounting board 11A also has a portion which is pushed when the plunger 10 is pushed. If the mounting board 11A is pushed, the plunger 10 slides relative to the tube 20, whereby the state of the cartridge changes from the state of Fig. 2A to the state of Fig. 2C. If the plunger 10 moves to the downstream side, the mounting board 11A comes into contact with the upper rim of the tube 20 (see Fig. 2C). That is, the interval between the mounting board 11A of the plunger 10 and the upper rim of the tube 20 becomes the sliding length of the plunger 10.

In the initial state, the rod-like portion 12 is positioned in the inside of the upper syringe 21 of the tube 20 and is separated from a lower syringe 22 (see Fig. 2A). If the plunger 10 slides relative to the tube 20, the rod-like portion 12 is inserted into the lower syringe 22 of the tube 20 and slides relative to the lower syringe 22 in the downstream direction while coming into contact with the inside of the lower syringe 22 (see Figs. 2B and 2C).

The cross-section of the rod-like portion 12 perpendicular to the longitudinal direction has a circular shape. However, the cross-sectional shape of the rod-like portion 12 may be circular, oval, or polygonal and is not particularly limited as long as the rod-like portion 12 can fit to the inner wall of the lower syringe 22 of the tube 20.

The seal 12A is formed in the end portion on the downstream side of the rod-like portion 12. If the seal 12A fits into the lower syringe 22, the liquid in the tube 20 on the downstream side is prevented from flowing back toward the upper syringe 21. Then, if the plunger 10 is pushed from the state of Fig. 2B to the state of Fig. 2C, the liquid in the tube 20 is pushed out from the downstream side by the amount corresponding to the volume in which the seal 12A slides inside the lower syringe 22.

The volume in which the seal 12A slides inside the lower syringe 22 (the amount of the liquid in the tube 20 pushed out from the downstream side) is greater than the total volume of the elution solution plug 47 and a third oil plug 48 in the tube 20. With this, the liquid in the tube 20 can be pushed out such that the elution solution 47 does not remain in the tube 20.

The material of the plunger 10 is not particularly limited and can be made of, for example, polypropylene, polyethylene, cycloolefin polymer (for example, ZEONEX (Registered Trademark) 480R), heat-resistant glass (for example, PYREX (Registered Trademark) glass), or a composite material thereof. The material of the plunger 10 may be the same as the material of the tank 3. The cylindrical portion 11 and the rod-like portion 12 of the plunger 10 may be formed of the same material integrally, or may be formed of different materials. Here, the cylindrical portion 11 and the rod-like portion 12 are separately molded with resin, and the cylindrical portion 11 and the rod-like portion 12 are bonded to each other through the ribs 13, whereby the plunger 10 is formed.

The plunger 10 accommodates oil 42 and a first washing solution 43 in advance in the inside thereof. The specific gravity of the oil 42 in the plunger 10 is smaller than that of the first washing solution 43. Therefore, when the tank 3 is attached to the cartridge main body 9, if the cartridge main body 9 is made to stand such that the mounting board 11A of the plunger 10 becomes the upper side, as shown in Fig. 2A, the oil 42 is disposed between the liquid in the tank 3 and the first washing solution 43 of the cartridge main body 9. As the oil 42, 2CS silicone oil is used, and as the first washing solution 43, 8M guanidine hydrochloride and 0.7% Triton X-100 are used.

The first washing solution 43 may be a liquid which undergoes phase separation when being mixed with any of the oil 42 and oil 44. It is preferable that the first washing solution 43 is water or a low salt-concentration aqueous solution, and the low salt-concentration aqueous solution is preferably a buffer solution. The salt concentration of the low salt-concentration aqueous solution is preferably equal to or less than 100 mM, more preferably equal to or less than 50 mM, and most preferably equal to or less than 10 mM. The lower limit of the low salt-concentration aqueous solution is not particularly limited, but is preferably equal to or greater than 0.1 mM, more preferably equal to or greater than 0.5 mM, and most preferably equal to or greater than 1 mM. This solution may also contain a surfactant, such as Triton, Tween, or SDS, and pH thereof is not particularly limited. The type of salt for making the buffer solution is not particularly limited, and salts, such as TRIS, HEPES, PIPES, and phosphoric acid, are preferably used. It is preferable that the washing solution contains alcohol in such an amount that does not hinder the adsorption of nucleic acid onto carriers, a reverse transcription reaction, a PCR reaction, and the like. In this case, the concentration of alcohol is not particularly limited, but the lower limit of the concentration of alcohol is preferably equal to or greater than 50%, more preferably equal to or greater than 60%, and most preferably equal to or greater than 70%. The upper limit of the concentration of alcohol is preferably equal to or less than 90%, more preferably equal to or less than 80%, and most preferably equal to or less than 70%. The type of alcohol is not particularly limited, and examples thereof include methanol, ethanol, propanol, acetonitrile, and the like. In terms of washing of nucleic acid and particles and the like onto which the nucleic acid is adsorbed, if at least one of the dissolution solution and the first washing solution contains alcohol, the washing effect can be enhanced. However, it is preferable that both of the dissolution solution and the first washing solution contain alcohol since the washing effect can be further increased.

The first washing solution 43 may contain a chaotropic agent. For example, if the first washing solution 43 contains guanidine hydrochloride, it is possible to wash particles and the like while maintaining or reinforcing the adsorption state of nucleic acid adsorbed onto the particles and the like. When the first washing solution contains guanidine hydrochloride, the concentration of this agent can be controlled, for example, to be equal to or greater than 3 mol/L and equal to or less than 10 mol/L, and preferably equal to or greater than 5 mol/L and equal to or less than 8 mol/L. If the concentration of guanidine hydrochloride is within the above range, it is possible to wash off foreign substances and the like while further stabilizing the adsorption of nucleic acid adsorbed onto particles and the like.

### 2-2. Tube

Hereinafter, the tube 20 will be described with reference to Figs. 2A to 2C.

The material of the tube 20 is not particularly limited, and it is more preferable to select transparent glass or resin since the inside can be observed. It is preferable to select a material which transmits a magnetic force or a nonmagnetic material as the material of the tube 20 since this makes it easy to move the magnetic particles by applying a magnetic force from the outside of the tube 20 so as to pass the magnetic particles through the tube 20. It is preferable that the material of the tube has heat resistance to a temperature of at least 100°C since a heater (a heater for elution 65A or a high temperature-side heater 65B described below) is disposed near the tube. Examples of the material satisfying these conditions include polypropylene, polyethylene, cycloolefin polymer (for example, ZEONEX (Registered Trademark) 480R), heat-resistant glass (for example, PYREX (Registered Trademark) glass), and a composite material thereof, and polypropylene is preferably used. The material of the tube 20 may be the same as the material of the tank 3 or the plunger 10.

The tube 20 has the shape of a cylinder through which a liquid can flow in the longitudinal direction. The tube 20 has the upper syringe 21, the lower syringe 22, and a capillary 23, and the inner diameter of each of these portions is different in a stepwise manner.

The upper syringe 21 has the shape of a cylinder through which a liquid can flow in the longitudinal direction. The cylindrical portion 11 of the plunger 10 slidably comes into contact with the inside of the inner wall of the upper syringe 21. The upper syringe 21 functions as a syringe for the cylindrical portion 11 of the plunger 10.

The lower syringe 22 has the shape of a cylinder through which a liquid can flow in the longitudinal direction. The seal 12A of the rod-like portion 12 of the plunger 10 can slidably fit to the inner wall of the lower syringe 22. The lower syringe 22 functions as a syringe for the rod-like portion 12 of the plunger 10.

The capillary 23 has the shape of a capillary tube through which a liquid can flow in the longitudinal direction. The inner diameter of the capillary 23 has such a size that enables the liquid to be maintained in the form of a plug. Here, the inner diameter is 1.0 mm. The inner diameter of the terminal (the terminal on the downstream side of the tube 20) of the capillary 23 is 0.5 mm which is smaller than the above-described diameter. The inner diameter of the terminal of the capillary 23 is set to be smaller than the diameter (1.5 mm to 2. 0 mm) of the elution solution, which will be described below, in the form of a droplet. Accordingly, when the elution solution plug 47 is pushed out from the terminal of the capillary 23, the elution solution in the form of a droplet can be inhibited from being adhered to the terminal of the capillary 23 or flowing back into the capillary 23.

The capillary 23 may have a cavity in the inside thereof and have the shape of a cylinder through which a liquid can flow in the longitudinal direction. The capillary 23 may be curved in the longitudinal direction, but it is preferable that the capillary 23 is straight. The internal cavity of the tube is not particularly limited in terms of size and shape as long as a liquid can be maintained in the form of a plug inside the tube. The size of the internal cavity of the tube or the shape of a cross-section thereof perpendicular to the longitudinal direction may be changed along the longitudinal direction of the tube.

The shape of the cross-section of the tube that is perpendicular to the longitudinal direction of the exterior of the tube is not limited. The thickness (a distance between the sidewall of the internal cavity and the exterior surface) of the tube is not particularly limited. When the tube has the shape of a cylinder, the inner diameter (the diameter of a circle which is a cross-section perpendicular to the longitudinal direction of the internal cavity) can be set to, for example, 0.5 mm to 2 mm. If the inner diameter of the tube is within the above range, it is easy to form a plug of a liquid within a wide range of tube materials and liquid types. It is preferable that the tube is tapered toward the tip, and the diameter of the tip can be set to 0.2 mm to 1 mm. If the inner diameter of the terminal of the capillary 23 (the diameter of the opening of the capillary 23) is set to be small, it is possible to inhibit the elution solution 47, which has been formed in a droplet in the PCR container 30, from being adsorbed onto the opening of the capillary 23 and becoming inseparable. However, if the inner diameter of terminal of the capillary 23 is too small, a large number of small droplets of the elution solution 47 are formed. In the capillary 23, if the diameter of a portion other than the terminal is made small just like the terminal, it is not desirable since the cartridge 1 will be lengthened to secure the volume of each plug.

In the inside of the capillary 23, a first oil plug 44, the washing solution plug 45, a second oil plug 46, the elution solution plug 47, and the third oil plug 48 are provided in order from the upstream side. That is, oil plugs are disposed on both sides of a water-soluble plug (the washing solution plug 45 or the elution solution plug 47).

The "plug" means a liquid when a certain liquid occupies a section in the tube. For example, in Figs. 2A to 2C, a liquid held in the form of a column inside a capillary 23 is called a "plug". Oil undergoes phase separation from other liquids (oil is not mixed with other liquids). Therefore, a plug formed of oil functions to prevent water-soluble plugs on both sides of the tube from being mixed with each other. Although it is preferable that air bubbles or other liquids are not present in a plug or between plugs, air bubbles or other liquids may be present as long as the magnetic beads 7 can pass through the plug.

In the upper syringe 21 and the lower syringe 22 on the upstream side from the first oil plug 44, the oil 42 and the washing solution 43 are accommodated in advance (see Fig. 2A). The inner diameter of the upper syringe 21 and the lower syringe 22 is greater than the inner diameter of the capillary 23. In the upper syringe 21 and the lower syringe 22, the liquid (the oil 42 and the washing solution 43) cannot be maintained in the form of a column just like a plug. However, since the first oil plug 44 is held in the form of a plug by the capillary 23, the oil constituting the first oil plug 44 is inhibited from moving to the upstream side.

The type of oil used herein is not particularly limited, and mineral oil, silicone oil (2CS silicone oil), plant oil, or the like can be used. If oil having higher viscosity is used, when the nucleic acid-binding solid-phase carriers are moved in the interface between the oil and the upper plug, a "wiping effect" produced by the oil can be enhanced. With this, when the nucleic acid-binding solid-phase carriers are moved from the upper plug to the plug formed of the oil, it is possible to make it more difficult for water-soluble components, which are adhered to the nucleic acid-binding solid-phase carriers, to be brought into the oil.

In the embodiment of the invention, at least one of the oil plugs in the tube becomes the plug of the first liquid, and contains oil and the first additive selected from a group consisting of carbinol-modified silicone resin, carboxyl-modified silicone resin, amino-modified silicone resin, polyether-modified silicone resin, silanol-modified silicone resin, and fluoro-modified silicone resin. In particular, it is preferable that the first additive is selected from a group consisting of X-22-160AS, X-22-3701E, KF-857, KF-859, KF-862, KF-867, KF-6017, KF-8005 (manufactured by Shin-Etsu Silicone Co., Ltd.), SS4230, SS4267, XF42-C5196, XF42-C5197, and XS66-C1191 (manufactured by Momentive Co. , Ltd.). The concentration of the first additive is higher than the concentration of the second additive containing the second liquid in the PCR container 30. The first additive and the second additive may be the same or different. It is preferable that one or more oil plugs from the elution solution plug 47 toward the PCR container 30 side become the plug of the first liquid. In this case, it is preferable to provide a plug which is formed of pure water or the like and is not mixed with oil, or oil mixing prevention means, such as a valve for preventing the downstream end of the capillary 23 from flowing back, such that the plug of the first liquid is not mixed with the second liquid in the PCR container 30.

As described below, the concentration of the additive in the PCR container 30 after the plug of the first liquid containing the first additive is ejected to the PCR container 30, that is, the ratio of the volume which is obtained by adding the volume of the second additive to the volume of the first additive when the plug of the first liquid is ejected to the PCR container 30, to the volume, which is obtained by adding the volume of the second liquid to the volume of the first liquid in the PCR container 30 is preferably equal to or greater than 1% (v/v) and equal to or less than 50% (v/v), and more preferably equal to or greater than 2% (v/v) and equal to or less than 20% (v/v), and still more preferably 5% (v/v). In order to obtain such a concentration, the concentrations of the additives contained in the first liquid in the plug of the tube 20 and the second liquid in the PCR container 30 may be determined.

The washing solution plug 45 may be formed of 5 mM of a tris-hydrochloric acid buffer solution which is a second washing solution. The washing solution plug 45 is preferably an acid solution, and particularly preferably an aqueous acid solution. The acid contained in the solution is not particularly limited, and an aqueous solution, such as citric acid, acetic acid, or glycine hydrochlorate, is preferably used. The solution may contain EDTA (ethylenediaminetetraacetic acid), a surfactant (Triton, Tween, or SDS), or the like. However, it is preferable that a solution does not substantially contain a chaotropic substance. The lower limit of pH is preferably equal to or greater than 1, more preferably equal to or greater than 2, still more preferably equal to or greater than 3, and most preferably equal to or greater than 4. The upper limit of pH is preferably equal to or less than 6, more preferably equal to or less than 5, and most preferably equal to or less than 4. If this first washing solution is used, even if nucleic acid or particles onto which the nucleic acid is adsorbed come into contact with a solution containing alcohol on the upstream side of the second washing solution, that is, even when the nucleic acid is extracted by a dissolution solution containing alcohol or the particles onto which the nucleic acid is adsorbed are washed by a washing solution containing alcohol, it is possible to efficiently wash the particles and the like, onto which the nucleic acid is adsorbed, by the second washing solution, to prevent alcohol from being brought toward the downstream, so-called carryover of alcohol, and to prevent inhibition of an enzyme reaction by alcohol.

The washing solution plug 45 may be constituted by a plurality of plugs divided by oil plugs. When the washing solution plug 45 is made of a plurality of plugs, the liquids of the respective plugs may be the same or different from one another. If at least one of the plugs is a plug of a washing solution, the liquids of other plugs are not particularly limited. However, it is preferable that all plugs are washing solutions. The number of divided plugs of the washing solution plug 45 can be appropriately set in consideration of, for example, the length of the tube 20 or the subject to be washed. At this time, it is preferable that a washing solution closest to the elution solution is an acid solution described in the washing solution plug 45, and other washing solutions contain alcohol as described in the first washing solution 43.

The elution solution 47 refers to the liquid that causes the nucleic acid adsorbed onto the nucleic acid-binding solid-phase carriers to be eluted in the solution from the carriers to cause a reverse transcription reaction and a polymerase reaction. Therefore, the elution solution 47 may be water or a buffer and may be prepared in advance such that the elution solution 47, in which the nucleic acid has been eluted, is directly used as a buffer solution for use in a reverse transcription reaction and a polymerase reaction. The salt which makes the elution solution be a buffer solution is not particularly limited as long as the salt does not hinder an enzyme reaction, and salts, such as TRIS, HEPES, PIPES, and phosphoric acid, are preferably used. For a reverse transcription reaction, the elution solution 47 may contain a reverse transcriptase, dNTP, and a primer for a reverse transcriptase (oligonucleotide). It is preferable that the elution solution 47 contains bovine serum albumin (BSA) or gelatin as a reaction hindrance inhibitor. The solvent is preferably water and more preferably an organic solvent, such as ethanol or isopropyl alcohol and a solvent which does not substantially contain a chaotropic substance.

The concentration of dNTP or salt contained in the elution solution may be appropriately set depending on the enzyme to be used in consideration of the concentration of dNTP or salt in the freeze-dried nucleic acid amplification reaction reagent. Generally, the concentration of dNTP is 10 to 1000 µM and preferably 100 to 500 µM, the concentration of Mg²⁺ is 1 to 100 mM and preferably 5 to 10 mM, and the concentration of Cl⁻ is 1 to 2000 mM and preferably 200 to 700 mM. The total ion concentration is not particularly limited, and is greater than 50 mM, preferably greater than 100 mM, more preferably greater than 120 mM, still more preferably greater than 150 mM, and even more preferably greater than 200 mM. The upper limit thereof is preferably equal to or less than 500 mM, more preferably equal to or less than 300 mM, and still more preferably equal to or less than 200 mM. The oligonucleotides for a primer are used at a concentration of 0.1 to 10 µM and preferably 0.1 to 1 µM. If the concentration of BSA or gelatin is equal to or less than 1 mg/mL, the reaction hindrance inhibitory effect is diminished, and if the concentration of BSA or gelatin is equal to or greater than 10 mg/mL, the reverse transcription reaction or the following enzyme reaction may be hindered. Therefore, the concentration is preferably 1 to 10 mg/mL. When gelatin is used, the source thereof is not particularly limited, and examples thereof include cowhide, pig hide, and beef bones. When gelatin is not easily dissolved, gelatin may be dissolved by heating.

The volume of the elution solution plug 47 is not particularly limited and can be appropriately set based on an index, such as the amount of particles and the like onto which nucleic acid is adsorbed. For example, when the volume of the particles and the like is 0.5 µL, it should suffice that the volume of the elution solution plug 47 is equal to or greater than 0.5 µL. The volume is preferably equal to or greater than 0.8 µL and equal to or less than 5 µL, and more preferably equal to or greater than 1 µL and equal to or less than 3 µL. If the volume of the elution solution plug 47 is within the above range, it is possible to cause the nucleic acid to be sufficiently eluted from the carriers, for example, even if the volume of the nucleic acid-binding solid-phase carriers is set to 0.5 µL.

The downstream portion of the capillary 23 is inserted into the PCR container 30. As a result, if the elution solution plug 47 in the tube 20 is pushed out from the tube 20, the elution solution 47 can be introduced into the PCR container 30.

A cyclic convexity of the outer wall of the capillary 23 comes into contact with the inner wall of the PCR container 30, whereby an upper seal portion is formed. The outer wall of the capillary 23 on the downstream side from the upper seal portion comes into contact with the inner wall of the PCR container 3 0, whereby a lower seal portion is formed. The upper seal portion and the lower seal portion will be described below.

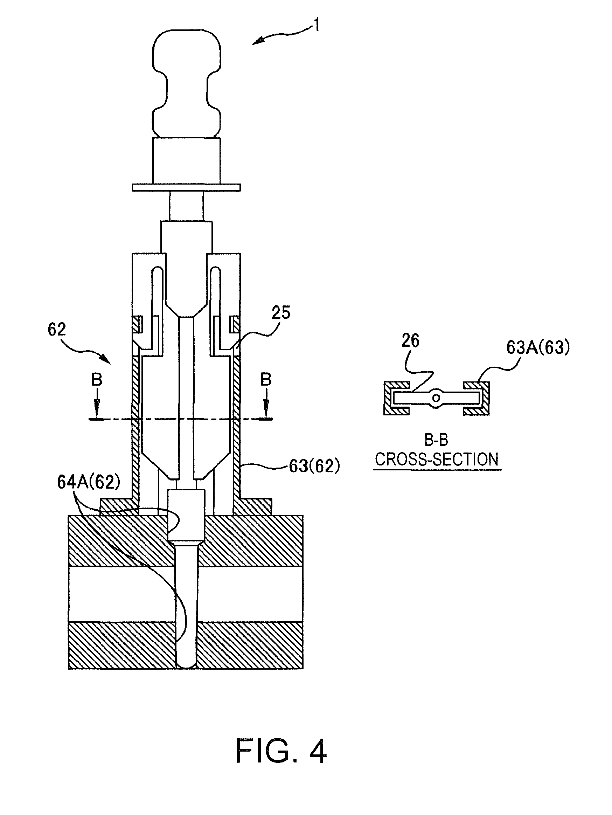

The tube 20 further has the fixing claw 25 and the guide plate 26. Fig. 4 is a diagram illustrating the fixing claw 25, the guide plate 26, and the mounting portion 62.

The fixing claw 25 is a member for fixing the cartridge 1 to the mounting portion 62. When the cartridge 1 is inserted into the mounting portion 62 until the fixing claw 25 is hooked on, the cartridge 1 is fixed to a normal position of the mounting portion 62. In other words, when the cartridge 1 is in an abnormal position of the mounting portion 62, the fixing claw 25 is not hooked on the mounting portion 62.

The guide plate 26 is a member for guiding the cartridge 1 when the cartridge 1 is mounted on the mounting portion 62 of the PCR device 50. A guide rail 63A is formed in the mounting portion 62 of the PCR device 50. The cartridge 1 is inserted into and fixed to the mounting portion 62 while being guided along the guide rail 63A by the guide plate 26 of the tube 20. The cartridge 1 is long. However, since the cartridge 1 is inserted into the mounting portion 62 under the guidance of the guide plate 26, it is easy to fix the cartridge 1 to a normal position of the mounting portion 62.

The fixing claw 25 and the guide plate 26 are plate-like members protruding from the right and left of the capillary 23. When the magnetic beads 7 in the tube 20 are moved by using a magnet, the magnet approaches the tube from the direction perpendicular to the plate-like fixing claw 25 or the guide plate 26. With this, the distance between the magnet and the magnetic beads 7 in the tube 20 can be shortened. However, the fixing claw 25 and the guide plate 26 may have other shapes as long as the distance between the magnet and the magnetic beads 7 in the tube 20 is shortened.

### 2-3. Nucleic Acid Amplification Reaction Container

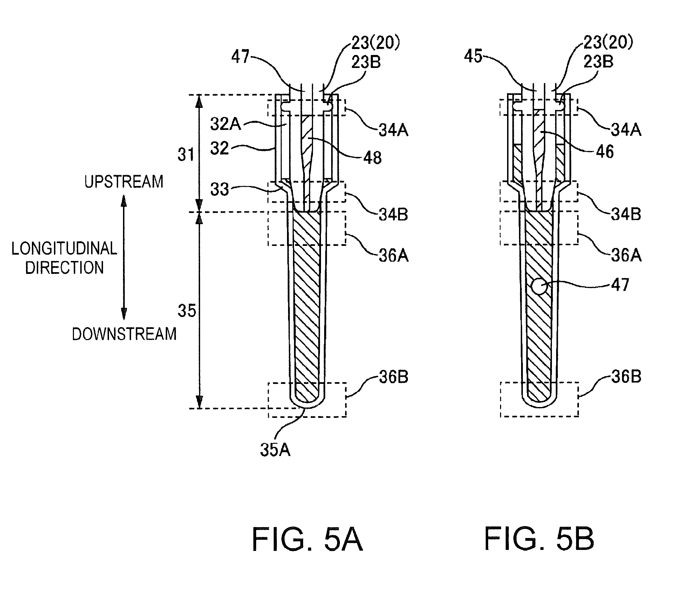

Figs. 5A and 5B are diagrams illustrating the periphery of the PCR container 30 which constitutes a nucleic acid amplification reaction container. Fig. 5A is a diagram illustrating the initial state. Fig. 5B is a diagram illustrating the state after the plunger 10 is pushed. Hereinafter, the PCR container 30 will be described also referring to Figs. 2A to 2C.

The type of the PCR container 30 is not particularly limited and for example, a small tube of 0.2 mL to 1.5 mL which can be directly used in a nucleic acid amplifier, such as a PCR device, is preferably used.

The material of the PCR container 30 is not particularly limited, and a material which has little adsorption of nucleic acid or protein and does not hinder an enzyme reaction by polymerase or the like is preferable used. It is preferable that the material of the PCR container 30 has heat resistance to a temperature of at least 100°C since the high temperature-side heater 65B is disposed near the PCR container 30. It is preferable to select a transparent or semitransparent material as the material of the PCR container 30 since this makes it easy to perform fluorescence measurement (fluorescence intensity measurement). However, the entire region of the PCR container 30 does not need to be transparent or semitransparent, and at least the site (for example, a bottom 35A of the PCR container 30) facing the fluorescence measuring instrument 55 may be transparent or semitransparent. Examples of the material satisfying these conditions include polypropylene, polyethylene, cycloolefin polymer (for example, ZEONEX (Registered Trademark) 480R), heat-resistant glass (for example, PYREX (Registered Trademark) glass), and a composite material thereof, and polypropylene is preferably used. The material of the PCR container 30 may be the same as the material of the tank 3 or the plunger 10. It is preferable that the end portion opposite to the side communicating with the tube 20 has a tapered shape, and for example, a shape in which the cross-sectional area thereof decreases toward the leading end. Accordingly, when the elution solution 47 in the form of a droplet precipitates, the elution solution 47 can reliably reach the freeze-dried nucleic acid amplification reaction reagent.

It is preferable that the PCR container 30 is filled with the second liquid, and a leading end on the side on which a fifth plug of the tube is disposed is in contact with the second liquid held in the PCR container 30. The type of oil contained in the second liquid is not particularly limited, and mineral oil, silicone oil (2CS silicone oil), plant oil, or the like can be used. It is preferable that the oil contained in the second liquid is the same type as oil in the tube 20. The viscosity of the oil is not particularly limited and is preferably small. The viscosity of the oil is preferably equal to or less than 90 cs, more preferably equal to or less than 70 cs, still more preferably equal to or less than 50 cs, and even more preferably equal to or less than 30 cs. Accordingly, as described below, the dissolution solution can precipitate more smoothly. The oil may contain a second additive selected from a group consisting of carbinol-modified silicone resin, carboxyl-modified silicone resin, amino-modified silicone resin, polyether-modified silicone resin, silanol-modified silicone resin, and fluoro-modified silicone resin. In particular, it is preferable that the second additive is selected from a group consisting of X-22-160AS, X-22-3701E, KF-857, KF-859, KF-862, KF-867, KF-6017, KF-8005 (manufactured by Shin-Etsu Silicone Co. , Ltd.), SS4230, SS4267, XF42-C5196, XF42-C5197, and XS66-C1191 (manufactured by Momentive Co., Ltd.). The concentration of the second additive is lower than the concentration of the first additive contained in the first liquid in the tube 20. The second liquid preferably has a low additive concentration, and most preferably, contains no additive. That is, the concentration of the second additive may be 0. The reason for the concentration of the second additive being low is that the second additive may hinder the activity of the nucleic acid amplification reaction reagent in the nucleic acid amplification reaction container, and in particular, as described below, when the freeze-dried nucleic acid amplification reaction reagent is held in the PCR container 30, the second additive inactivates the nucleic acid amplification reaction reagent during storage of the cartridge. It is preferable that the second additive is the same type as the first additive contained in the first liquid in the tube 20.

In the container, it is preferable that the nucleic acid amplification reaction reagent is held in the second liquid. The nucleic acid amplification reaction reagent may be held in the form of a liquid, but is preferably held in a freeze-dried state from the viewpoint of stability during storage. It is preferable that the freeze-dried nucleic acid amplification reaction reagent is not dissolved in the second liquid. The nucleic acid amplification reaction reagent contains at least DNA polymerase and dNTP, but preferably contains a primer for a nucleic acid amplification reaction and/or a probe for a nucleic acid amplification reaction. The freeze-dried nucleic acid amplification reaction reagent may contain a reverse transcriptase. In this case, a primer for reverse transcription may be contained separately from the primer for a nucleic acid amplification reaction, or may be used in common with the primer for a nucleic acid amplification reaction.

It is preferable that the nucleic acid amplification reaction reagent contains a surfactant. The surfactant is not particularly limited, and examples of the surfactant include NP40, Triton-X100, Tween20, and the like. The concentration of the surfactant is not particularly limited, and the surfactant is preferably used in such a concentration that does not hinder a nucleic acid amplification reaction. The concentration of the surfactant may be 0.001% to 0.1%, is preferably 0.002% to 0.02%, and is most preferably 0.005% to 0.01%. The surfactant may be brought from a stock solution of DNA polymerase or a surfactant solution may be separately added to the nucleic acid amplification reaction reagent.

The DNA polymerase is not particularly limited, and a heat resistant enzyme or an enzyme for PCR is preferably used. For example, there are an extremely large number of commercially available products, such as Taq polymerase, Tfi polymerase, Tth polymerase, and enzymes obtained by modifying the above enzymes. However, it is preferable to use DNA polymerase which can be subjected to hot start. The reverse transcriptase is not particularly limited, and for example, reverse transcriptase derived from avian myeloblast virus, ras-associated virus type 2, mouse molony murine leukemia virus, and human immunodeficiency virus type 1 can be used. However, it is preferable to use a heat-resistant enzyme.

The concentration of dNTP or salt may be appropriately set depending on the enzyme to be used. Generally, the concentration of dNTP is 10 to 1000 µM and preferably 100 to 500 µM, the concentration of Mg²⁺ is 1 to 100 mM and preferably 5 to 10 mM, and the concentration of Cl⁻ is 1 to 2000 mM and preferably 200 to 700 mM. The total ion concentration is not particularly limited, and is greater than 50 mM, preferably greater than 100 mM, more preferably greater than 120 mM, still more preferably greater than 150 mM, and even more preferably greater than 200 mM. The upper limit thereof is preferably equal to or less than 500 mM, more preferably equal to or less than 300 mM, and still more preferably equal to or less than 200 mM. The oligonucleotides for a primer are used at a concentration of 0.1 to 10 µM and preferably 0.1 to 1 µM.

The freeze-drying of the nucleic acid amplification reaction reagent may be performed by a method which is generally performed. For example, a nucleic acid amplification reaction reagent for single reaction is put in a buffer for a nucleic acid amplification reaction in the PCR container 30, is quickly frozen, and is maintained for a predetermined time under low pressure. The amount of the buffer for a nucleic acid amplification reaction is not particularly limited, but is 5 µL, preferably 2.5 µL, and more preferably 1.6 µL. The less the amount of the buffer, the smaller and harder the nucleic acid amplification reaction reagent is fixed to the bottom of the container. The temperature during the freeze-drying is not particularly limited, and is preferably -10°C to -160°C and most preferably about -80°C. It is preferable that the freeze-dried nucleic acid amplification reaction reagent is a porous material in a sponge-like or cake-like form having many micropores (pores) in which air bubbles are accumulated. Accordingly, solubility is improved. If the nucleic acid amplification reaction reagent is quickly frozen, the micropores further decrease, and preservability is further improved. The average void diameter (for example, the diameter of a pore in a cross-section is measured a given number of times using a microscope, and the average value thereof is taken) of the pores is equal to or less than 20 µm. The pressure in low pressure is not particularly limited and is preferably equal to or less than 100 mmHg and most preferably equal to or less than 20 mmHg. The time for which the maintained under low pressure is not particularly limited and is preferably 2 to 24 hours and most preferably about 8 hours. It is preferable that the amount of a nucleic acid amplification reaction reagent solution for freeze-drying the nucleic acid amplification reaction reagent is less than the amount of an aqueous solution for dissolving the nucleic acid amplification reaction reagent.

In this way, if the nucleic acid amplification reaction reagent is freeze-dried in the PCR container 30, the nucleic acid amplification reaction reagent is fixed to the bottom of the PCR container 30. Thereafter, the second liquid is added. In this case, it is preferable to add the second liquid such that the container is filled with the second liquid. The freeze-dried nucleic acid amplification reaction reagent is weak against moisture and cannot be preserved stably over a long period of time in a state where there is moisture in the second liquid. Therefore, it is preferable that the second liquid is dehydrated in advance by silica gel or the like, and the addition of the second liquid is performed inside a glove box or the like. It is preferable that the container is centrifuged lightly to remove air bubbles after the second liquid is added.

Before the second liquid is added to the freeze-dried nucleic acid amplification reaction reagent, dissolved wax is added and the second liquid is added after the wax is solidified, whereby it is possible to prevent the freeze-dried nucleic acid amplification reaction reagent from being diffused in the second liquid. Here, wax is an organic substance which is solid at room temperature and becomes liquid when being heated. It is preferable that the wax usable in the invention has a melting point equal to or higher than 31°C, preferably equal to or higher than 36°C, more preferably equal to higher than 41°C, and still more preferably equal to or higher than 46°C, and equal to or lower than 100°C, preferably equal to or lower than 90°C, more preferably equal to or lower than 80°C, and still more preferably equal to or lower than 70°C, and is made of neutral fat, higher fatty acid, hydrocarbon, and the like. The wax is not particularly limited, and for example, waxes derived from petroleum, such as paraffin and microcrystalline, waxes derived from animal, such as beeswax, wool wax, and spermacetic wax, and waxes derived from plant, such as carnauba, rocin, candelilla wax, and Japan wax, can be used. In addition, EL CHRISTA (Registered Trademark, IDEMITSU KOSAN Co., Ltd.), NISSAN ELEC TORR (Registered Trademark, NOF Corporation), POEM (Registered Trademark, RIKEN VITAMIN Co., Ltd.), RIKEMAL (Registered Trademark, RIKEN VITAMIN Co., Ltd.), NEOWAX (Registered Trademark, YASUHARA CHEMICAL Co., Ltd.), HIGH WAX (Registered Trademark, MITSUI CHEMICALS, Inc.), SILICON WAX (Registered Trademark, DOW CORNING TORAY Co. Ltd.), and the like can be used.

The PCR container 30 having the freeze-dried nucleic acid amplification reaction reagent is a container which receives the liquid pushed out from the tube 20 and accommodates the elution solution 47 during the thermal cycle process. Hereinafter, a structure example of the PCR container 30 will be described.

The PCR container 30 has a seal forming portion 31 and a flow path forming portion 35. The seal forming portion 31 is a portion into which the tube 20 is inserted and which inhibits the second liquid, which overflows from the flow path forming portion 35, from leaking to the outside. The flow path forming portion 35 is placed on the downstream side from the seal forming portion 31 and forms a flow path through which the elution solution 47 in the form of a droplet moves. The PCR container 30 is fixed to the tube 20 at two sites including an upper seal portion 34A and a lower seal portion 34B of the seal forming portion 31.

The seal forming portion 31 has an oil accommodating portion 32 and a stepped portion 33.

The oil accommodating portion 32 is a cylindrical portion and functions as a reservoir accommodating the second liquid which overflows from the flow path forming portion 35. There is a gap between the inner wall of the oil accommodating portion 32 and the outer wall of the capillary 23 of the tube 20, and this gap becomes an oil accommodating space 32A accommodating the second liquid which overflows from the flow path forming portion 35. The volume of the oil accommodating space 32A is greater than the volume in which the seal 12A of the plunger 10 slides in the lower syringe 22 of the tube 20.

The inner wall on the upstream side of the oil accommodating portion 32 comes into contact with the cyclic convexity of the tube 20, whereby the upper seal portion 34A is formed. The upper seal portion 34A is a seal which allows permeation of air while inhibiting the second liquid of the oil accommodating space 32A from leaking to the outside. In the upper seal portion 34A, a vent having such a size that does not allow the second liquid to leak due to the surface tension of the second liquid is formed. The vent of the upper seal portion 34A may be a gap between the convexity of the tube 20 and the inner wall of the oil accommodating portion 32, or may be a hole, groove, or notch formed in the convexity of the tube 20. The upper seal portion 34A may be formed of an oil absorbent absorbing oil.

The stepped portion 33 is a portion which is disposed on the downstream side of the oil accommodating portion 32 and shows a step difference. The inner diameter of the downstream portion of the stepped portion 33 is smaller than the inner diameter of the oil accommodating portion 32. The inner wall of the stepped portion 33 comes into contact with the outer wall on the downstream side of the capillary 23 of the tube 20. The inner wall of the stepped portion 33 comes into contact with the outer wall of the tube 20, whereby the lower seal portion 34B is formed. The lower seal portion 34B is a seal which allows the second liquid of the flow path forming portion 35 to flow to the oil accommodating space 32A while exhibiting resistance to the flow. Due to pressure loss of the lower seal portion 34B, the pressure in the flow path forming portion 35 becomes higher than the outside pressure. Therefore, even if the liquid in the flow path forming portion 35 is heated during the thermal cycle process, air bubbles are not easily formed in the liquid in the flow path forming portion 35.

The flow path forming portion 35 is a tubular portion and functions as a container forming a flow path through which the elution solution 47 in the form of a droplet moves. The flow path forming portion 35 is filled with the second liquid. The upstream side of the flow path forming portion 35 is closed by the terminal of the tube 20, and the terminal of the tube 20 is opened toward the flow path forming portion 35. The inner diameter of the flow path forming portion 35 is greater than the inner diameter of the capillary 23 of the tube 20, and is also greater than the outer diameter of a sphere of the liquid having the volume of the elution solution plug 47 which is formed in a sphere. It is desirable that the inner wall of the flow path forming portion 35 exhibits water repellency to such a degree that the water-soluble elution solution 47 does not adhere to the inner wall.

The upstream side of the flow path forming portion 35 is heated at a relatively high temperature (for example, about 95°C) by the high temperature-side heater 65B of the outside, thereby forming the high temperature region 36A. The downstream side of the flow path forming portion 35 is heated at a relatively low temperature (for example, about 60°C) by the low temperature-side heater 65C of the outside, thereby forming the low temperature region 36B. The bottom 35A (edge of the downstream side) of the PCR container 30 includes the low temperature region 36B. As a result, a temperature gradient is formed in the liquid in the flow path forming portion 35.

As shown in Fig. 5A, in the initial state, the flow path forming portion 35 of the PCR container 30 is filled with the second liquid. The interface of the second liquid is positioned on the comparatively downstream side from the oil accommodating space 32A. In the oil accommodating space 32A, the volume of the upstream side from the interface of the second liquid is greater than the volume in which the seal 12A of the plunger 10 slides in the lower syringe 22 of the tube 20.

As shown in Fig. 5B, when the plunger 10 is pushed, the liquid in the tube 20 is pushed out into the flow path forming portion 35. Since the second liquid in the tube 20 is pushed out into the flow path forming portion 35 which has already been filled with oil, gas does not flow into the flow path forming portion 35.

When the plunger 10 is pushed, first, the third oil plug 48 of the tube 20 flows into the flow path forming portion 35, and the inflow oil then flows into the oil accommodating space 32A from the flow path forming portion 35, whereby the oil interface of the oil accommodating space 32A ascends. At this time, due to pressure loss of the lower seal portion 34B, the pressure of a liquid in the flow path forming portion 35 increases. After the third oil plug 48 is pushed out from the tube 20, the elution solution plug 47 flows into the flow path forming portion 35 from the tube 20. Since the inner diameter of the flow path forming portion 35 is greater than the inner diameter of the capillary 23 and the elution solution 47 has specific gravity greater than oil, the elution solution 47, which has been in the form of a plug (form of a column) in the tube 20, becomes a droplet in the second liquid in the flow path forming portion 35 and precipitates inside the second liquid. In the initial state, the volume of the oil accommodating space 32A which is on the upstream side from the interface of the second liquid is greater than the volume in which the seal 12A of the plunger 10 slides in the lower syringe 22 of the tube 20. Therefore, the second liquid does not overflow from the oil accommodating space 32A. The precipitating elution solution 47 in the form of a droplet dissolves the nucleic acid amplification reaction reagent freeze-dried in the PCR container 30, and becomes an RT-PCR reaction solution or a PCR reaction solution.

### PCR Device 50

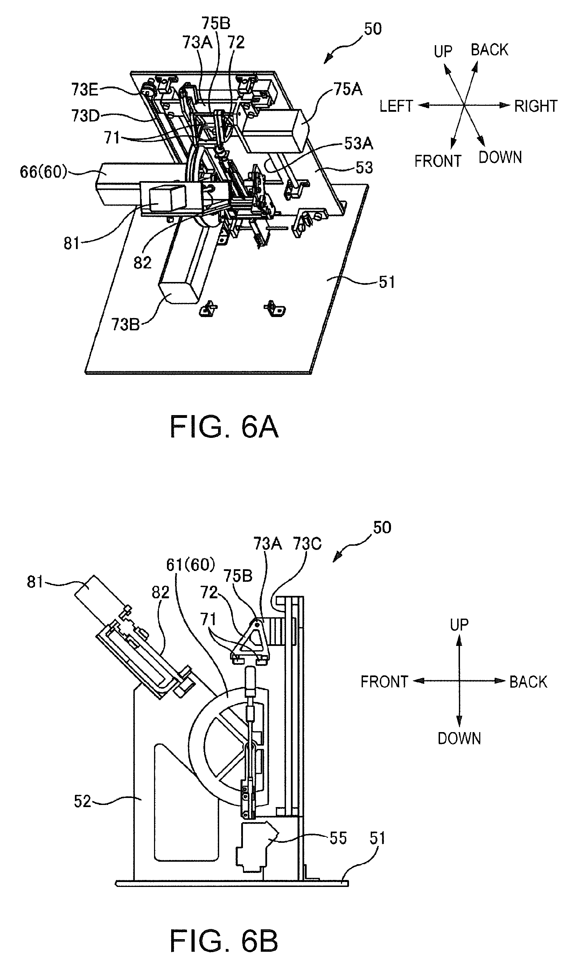

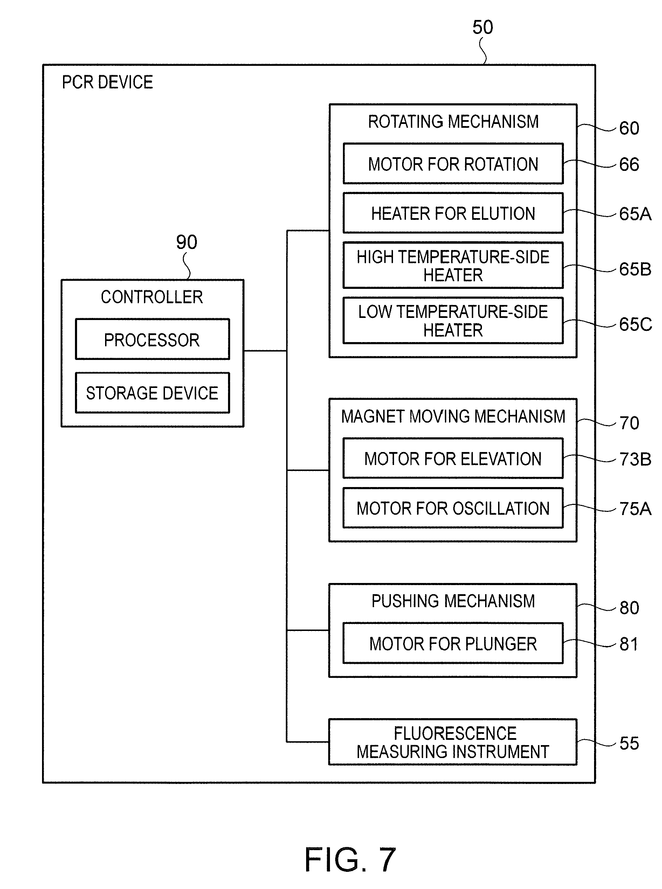

Fig. 6A is a perspective view showing the internal configuration of the PCR device 50. Fig. 6B is a side view showing the main configuration of the PCR device 50. Fig. 7 is a block diagram of the PCR device 50. The PCR device 50 is a device which performs a nucleic acid elution process and a thermal cycle process using the cartridge 1.

In the following description of the PCR device 50, the meanings of "up and down", "front and back", and "left and right" will be defined as shown in the drawing. When a base 51 of the PCR device 50 is horizontally disposed, a direction perpendicular to the base 51 is defined as an "up-down direction", and "up" and "down" will be defined according to the direction of gravity. The axial direction of the rotary shaft of the cartridge 1 is defined as a "right-left direction", and a direction perpendicular to the up-down direction and the right-left direction is defined as a "front-back direction". When the PCR device 50 is viewed from the rotary shaft of the cartridge 1, a cartridge insertion port 53A side is defined as "back", and a side opposite to "back" is defined as "front". When the PCR device 50 is viewed from the front side, the right side and the left side in the right-left direction are respectively defined as "right" and "left".

The PCR device 50 has a rotating mechanism 60, a magnet moving mechanism 70, a pushing mechanism 80, a fluorescence measuring instrument 55, and a controller 90.

### 1. Rotating Mechanism 60

The rotating mechanism 60 is a mechanism for rotating the cartridge 1 and the heater. When the cartridge 1 and the heater are moved upside down by the rotating mechanism 60, the elution solution 47 in the form of a droplet moves inside the flow path forming portion 35 of the PCR container 30, whereby the thermal cycle process is performed.

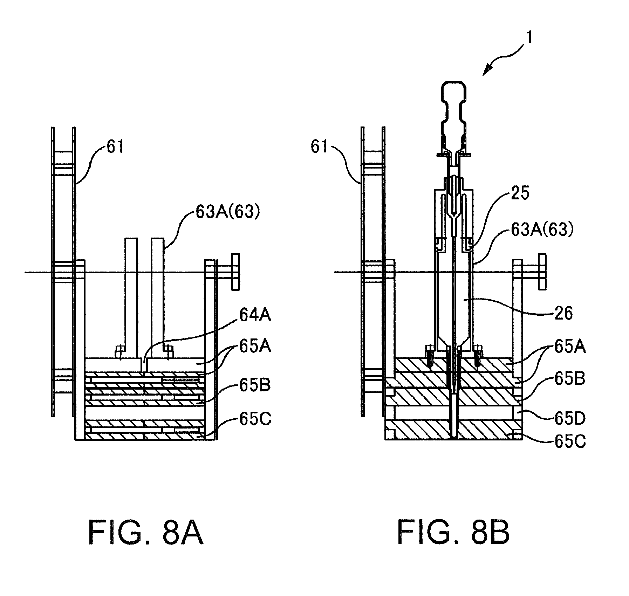

The rotating mechanism 60 has a rotary body 61 and a motor for rotation 66. Fig. 8A is a diagram illustrating the rotary body 61. Fig. 8B is a diagram illustrating the state where the cartridge 1 is mounted on the mounting portion 62 of the rotary body 61.

The rotary body 61 is a member rotatable around the rotary shaft. The rotary shaft of the rotary body 61 is supported on a support 52 fixed to the base 51. The rotary body 61 is provided with the mounting portion 62 on which the cartridge 1 is mounted and the heaters (the heater for elution 65A, the high temperature-side heater 65B, and the low temperature-side heater 65C). If the rotary body 61 rotates, the cartridge 1 can be moved upside down in a state where the positional relationship between the cartridge 1 and the heater is maintained. The motor for rotation 66 is a power source for rotating the rotary body 61. The motor for rotation 66 rotates the rotary body 61 to a predetermined position according to the instruction from the controller 90. A transmission mechanism, such as a gear, may be interposed between the motor for rotation 66 and the rotary body 61.

The rotary shaft of the rotary body 61 is positioned closer to the tube 20 than to the PCR container 30 of the cartridge 1. In other words, the rotary shaft of the rotary body 61 is positioned in the tube 20 of the cartridge 1 mounted on the mounting portion 62. This is because the tube 20 is longer than the PCR container 30, and therefore, if the center of the PCR container 30 is used as the rotary shaft (if the rotary shaft of the rotary body 61 is positioned in the PCR container), the size of the rotary body 61 will increase.

The mounting portion 62 is a portion on which the cartridge 1 is mounted. The mounting portion 62 has a fixing portion 63 in which a notch is formed. An insertion hole 64A formed in the heaters (the heater for elution 65A, the high temperature-side heater 65B, and the low temperature-side heater 65C) also functions as the mounting portion 62. When the fixing claw 25 of the cartridge 1 is hooked on the notch of the fixing portion 63 in a state where the PCR container 30 is inserted into the insertion hole 64A, the cartridge 1 is mounted on the rotary body 61 (see Fig. 4). Here, a portion of the heater also functions as the mounting portion 62, but the mounting portion 62 may be separated from the heater. The mounting portion 62 is indirectly fixed to the rotary body 61 through the heater for elution 65A. However, the mounting portion 62 may be directly disposed in the rotary body 61. The number of the cartridge 1 that can be mounted on the mounting portion 62 is not limited to one, and a plurality of cartridges 1 may be mounted on the mounting portion 62.

The fixing portion 63 of the mounting portion 62 functions as a tube fixing portion to which the tube 20 of the cartridge 1 is fixed. The insertion hole 64A functions as a PCR container fixing portion to which the PCR container 30 is fixed. With this, the long cartridge 1 including the tube 20 and the PCR container 30 is stably fixed to the mounting portion 62.

The guide rail 63A is formed in the fixing portion 63 along the up-down direction (see Fig. 4). The guide rail 63A guides the guide plate 26 of the cartridge 1 in the insertion direction while constraining the guide plate 26 in the front-back direction. While the guide plate 26 is being guided by the guide rail 63A, the cartridge 1 is inserted into the mounting portion 62. Therefore, the PCR container 30 of the cartridge 1 is guided to the insertion hole 64A, whereby the cartridge 1 is fixed to a normal position of the mounting portion 62.

The PCR device 50 has the high temperature-side heater 65B and the low temperature-side heater 65C, which are heaters for PCR, and the heater for elution 65A. Each of the heaters is constituted by a heating source (not shown) and a heat block. The heating source is, for example, a cartridge heater and is inserted into the heat block. The heat block is, for example, a metal with high heat conductivity, such as aluminum, and heats the liquid in the cartridge 1 with heat from the heating source while suppressing heat unevenness. It is desirable for the heat block to be a nonmagnetic substance to prevent the magnets 71, which move the magnetic beads 7, from being adsorbed onto the heat block.

The heater for elution 65A is a heater that heats the elution solution plug 47 of the cartridge 1. When the cartridge 1 is fixed to a normal position, the heater for elution 65A faces the elution solution plug 47 of the tube 20. For example, the heater for elution 65A heats the elution solution plug 47 to about 50°C, whereby liberation of nucleic acid from the magnetic beads is accelerated.

The high temperature-side heater 65B is a heater that heats the upstream side of the flow path forming portion 35 of the PCR container 30. When the cartridge 1 is fixed to a normal position, the high temperature-side heater 65B faces the upstream side (high temperature region 36A) of the flow path forming portion 35 of the PCR container 30. For example, the high temperature-side heater 65B heats the liquid of the upstream side of the flow path forming portion 35 of the PCR container 30 to about 90°C to 100°C.

The low temperature-side heater 65C is a heater that heats the bottom 35A of the flow path forming portion 35 of the PCR container 30. When the cartridge 1 is fixed to a normal position, the low temperature-side heater 65C faces the downstream side (the low temperature region 36B) of the flow path forming portion 35 of the PCR container 30. For example, the low temperature-side heater 65C heats the liquid in the low temperature region 36B of the PCR container 30 to about 50°C to 75°C.

A spacer 65D is disposed between the high temperature-side heater 65B and the low temperature-side heater 65C. The spacer 65D suppresses heat conduction between the high temperature-side heater 65B and the low temperature-side heater 65C. The spacer 65D is also used for accurately setting the distance between the high temperature-side heater 65B and the low temperature-side heater 65C. With this, a temperature gradient is formed in the liquid in the flow path forming portion 35 of the PCR container 30 by the high temperature-side heater 65B and the low temperature-side heater 65C.

In the heat block constituting each of the heater for elution 65A, the high temperature-side heater 65B, and the low temperature-side heater 65C, a through hole constituting the insertion hole 64A is formed. The outer wall of the bottom 35A of the PCR container 30 is exposed through the lower opening of the insertion hole 64A of the low temperature-side heater 65C. The fluorescence measuring instrument 55 measures the luminance of the elution solution 47 from the lower opening of the insertion hole 64A.

Each of the high temperature-side heater 65B and the low temperature-side heater 65C is provided with a temperature control device. Accordingly, the temperature can be set to a level suitable for each polymerase reaction.

### 2. Magnet Moving Mechanism 70

The magnet moving mechanism 70 is a mechanism moving the magnets 71. The magnet moving mechanism 70 causes the magnetic beads 7 in the cartridge 1 to be attracted to the magnets 71 and moves the magnets 71 such that the magnetic beads 7 move to the inside of the cartridge 1. The magnet moving mechanism 70 has a pair of magnets 71, an elevating mechanism 73, and an oscillating mechanism 75.

The magnets 71 are members attracting the magnetic beads 7. As the magnets 71, a permanent magnet, an electromagnet, and the like can be used. Here, a permanent magnet that does not generate heat or the like is used. The pair of magnets 71 is held in an arm 72 so as to face each other in the front-back direction in a state where the position thereof in the up-down direction is substantially the same. The respective magnets 71 can face each other on the front side or the back side of the cartridge 1 mounted on the mounting portion 62. The pair of magnets 71 can sandwich the cartridge 1 mounted on the mounting portion 62 therebetween in the front-back direction. If the magnets 71 face each other in a direction (herein, front-back direction) orthogonal to the direction (herein, right-left direction) in which the fixing claw 25 or the guide plate 26 of the cartridge 1 is disposed, the distance between the magnetic beads 7 in the cartridge 1 and the magnets 71 can be shortened.

The elevating mechanism 73 is a mechanism for moving the magnets 71 in the up-down direction. Since the magnets 71 attract the magnetic beads 7, if the magnets 71 are moved in the up-down direction according to the movement of the magnetic beads 7, the magnetic beads 7 in the cartridge 1 can be attracted in the up-down direction.

The elevating mechanism 73 has a carriage 73A moving in the up-down direction and a motor for elevation 73B. The carriage 73A is a member movable in the up-down direction and is guided so as to be movable in the up-down direction by a carriage guide 73C disposed in a sidewall 53 where the cartridge insertion port 53A is placed. The arm 72 holding a pair of magnets 71 is attached to the carriage 73A. Therefore, if the carriage 73A moves in the up-down direction, the magnets 71 move in the up-down direction. The motor for elevation 73B is a power source for moving the carriage 73A in the up-down direction. The motor for elevation 73B moves the carriage 73A to a predetermined position in the up-down direction according to the instruction from the controller 90. Although the motor for elevation 73B moves the carriage 73A in the up-down direction using a belt 73D and a pulley 73E, the motor for elevation 73B may move the carriage 73A in the up-down direction by other transmission mechanisms.

When the carriage 73A is at the uppermost position (retraction position), the magnets 71 are positioned above the cartridge 1. When the carriage 73A is at the retraction position, the elevating mechanism 73 does not come into contact with the cartridge 1 even if the cartridge 1 rotates. The elevating mechanism 73 can bring down the carriage 73A to a position where the magnets 71 face the reaction plug. Therefore, the elevating mechanism 73 can move the magnets 71 such that the magnetic beads 7 in the tank 3 move to the position of the reaction plug.

The oscillating mechanism 75 is a mechanism for oscillating the pair of magnets 71 in the front-back direction. When the pair of magnets 71 oscillates in the front-back direction, each of the magnets 71 becomes distant from the cartridge 1 by a different length. Since the magnetic beads 7 are attracted to the magnets 71 close to the cartridge 1, if the pair of magnets 71 oscillates in the front-back direction, the magnetic beads 7 in the cartridge 1 move in the front-back direction.

The oscillating mechanism 75 has a motor for oscillation 75A and a gear. The motor for oscillation 75A and the gear are disposed in the carriage 73A and can move in the up-down direction together with the carriage 73A. When the power of the motor for oscillation 75A is transmitted to the arm 72 through the gear, the arm 72 holding the magnets 71 rotates around an oscillating rotary shaft 75B relative to the carriage 73A. In order to prevent damage to the cartridge 1 due to contact between the magnets 71 and the cartridge 1, the oscillating mechanism 75 causes the magnets 71 to oscillate within a range in which the magnets 71 do not come into contact with the cartridge 1.

The oscillating rotary shaft 75B is a rotary shaft of the arm 72. The oscillating rotary shaft 75B is in parallel with the horizontal direction so as to cause the magnets 71 to oscillate in the front-back direction. When the oscillating rotary shaft 75B is viewed from the right or left, the oscillating rotary shaft 75B is disposed at a position depart from the cartridge 1 toward the front or back of the cartridge. Therefore, when the carriage 73A moves to the lower side, the contact between the cartridge 1 and the arm 72 can be avoided. If the magnets 71 can oscillate in the front-back direction, the oscillating rotary shaft 75B may be a shaft in parallel with the up-down direction.

### 3. Pushing Mechanism 80

The pushing mechanism 80 is a mechanism for pushing the plunger 10 of the cartridge 1. When the plunger 10 is pushed by the pushing mechanism 80, the elution solution plug 47 and oil plug of the cartridge 1 are pushed out to the PCR container 30, whereby the elution solution 47 in the form of a droplet is formed in the second liquid of the PCR container 30.

The pushing mechanism 80 has a motor for a plunger 81 and a rod 82. The motor for a plunger 81 is a source of power for moving the rod 82. The rod 82 is a member for pushing the mounting board 11A of the plunger 10 of the cartridge 1. The reason for pushing the mounting board 11A instead of the tank 3 of the cartridge 1 is that the tank 3 is made of flexible resin which is dilatable. When the tank 3 is not deformed, the pushing mechanism 80 may push the tank 3 to push the plunger 10.

The plunger 10 is pushed by the rod 82, not in the up-down direction but in a direction inclined from the up-down direction by 45 degrees. Accordingly, when the plunger 10 is pushed by the pushing mechanism 80, in the PCR device 50, the rotary body 61 is rotated by 45 degrees to match the longitudinal direction of the cartridge 1 with the movement direction of the rod 82, and then the rod 82 is moved. Since the rod 82 pushes the plunger 10 in the direction inclined from the up-down direction by 45 degrees, it is easy to dispose the pushing mechanism 80 such that this mechanism becomes free from interference of the elevating mechanism 73. Since the rod 82 pushes the plunger 10 in the direction inclined from the up-down direction by 45 degrees, the size of the PCR device 50 can be reduced in the up-down direction.

### 4. Fluorescence Measuring Instrument 55

The fluorescence measuring instrument 55 has an excitation light source which irradiates excitation light onto the elution solution 47 of the PCR container 30, and a fluorophotometer which measures intensity of fluorescence emitted from the elution solution 47. The fluorescence measuring instrument 55 is disposed below the rotary body 61 so as to face the bottom 35A of the PCR container 30 of the cartridge 1. The fluorescence measuring instrument 55 measures intensity of fluorescence emitted from the elution solution 47 at the bottom 35A of the PCR container 30 from the lower opening of the insertion hole 64A of the low temperature-side heater 65C.

### 5. Controller 90

The controller 90 is a control device for controlling the PCR device 50. The controller 90 has, for example, a processor, such as CPU, and a storage device, such as a ROM or a RAM. The stored device stores various programs and data. The storage device provides an area for running the programs. When the processor executes a program stored in the storage device, various processes are performed.

For example, the controller 90 controls the motor for rotation 66 to rotate the rotary body 61 to a predetermined rotation position. The rotating mechanism 60 is provided with a rotation position sensor (not shown), and the controller 90 drives and stops the motor for rotation 66 according to a detection result of the rotation position sensor.

The controller 90 controls the heaters (the heater for elution 65A, the high temperature-side heater 65B, and the low temperature-side heater 65C) such that each heater generates heat. The heat block constituting the heater is provided with a temperature sensor (not shown), and the controller 90 controls ON and OFF of the cartridge heater according to a detection result of the temperature sensor.

The controller 90 controls the motor for elevation 73B to move the magnets 71 in the up-down direction. The PCR device 50 is provided with a position sensor (not shown) which detects the position of the carriage 73A, and the controller 90 drives and stops the motor for elevation 73B according to a detection result of the position sensor.

The controller 90 controls the motor for oscillation 75A to oscillate the magnet 71 in the front-back direction. The PCR device 50 is provided with a position sensor which detects the position of the arm 72 holding the magnets 71, and the controller 90 drives and stops the motor for oscillation 75A according to a detection result of the position sensor.

The controller 90 controls the fluorescence measuring instrument 55 to measure fluorescence intensity of the elution solution 47 of the PCR container 30. The controller 90 causes the fluorescence measuring instrument 55 to perform measurement when the fluorescence measuring instrument 55 faces the bottom 35A of the PCR container 30 of the cartridge 1. A measurement result is stored in the storage device.

### Description of Operation

### 1. Operation for Mounting Cartridge 1

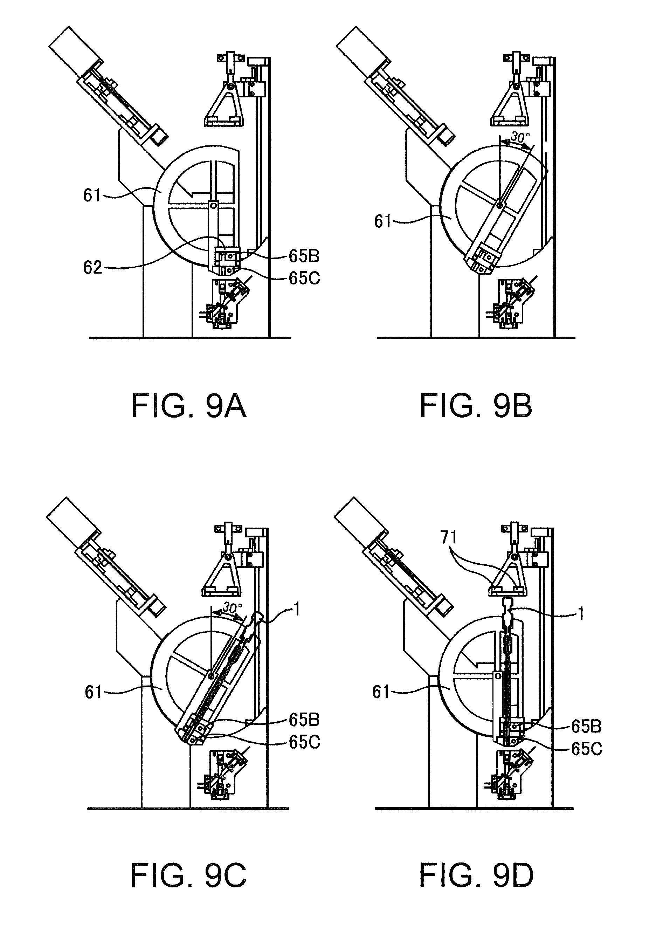

Figs. 9A to 9D are diagrams illustrating the state of the PCR device 50 when mounting the cartridge 1. Fig. 9A is a diagram illustrating an initial state where the cartridge 1 is mounted. Fig. 9B is a diagram illustrating a standby state. Fig. 9C is a diagram illustrating the state immediately after the cartridge 1 is mounted. Fig. 9D is a diagram illustrating an initial state in the state where the cartridge 1 is mounted.

As shown in Fig. 9A, in the initial state before the cartridge 1 is mounted, the mounting direction of the mounting portion 62 is in the up-down direction. In the following description, the rotation position of the rotary body 61 in this state is taken as a reference (0 degrees), and if the rotary body 61 rotates in a counter clockwise direction when viewed from the right, the direction is referred to as a positive direction.

As shown in Fig. 9B, the controller 90 drives the motor for rotation 66 to rotate the rotary body 61 by -30°. In this state, an operator inserts the cartridge 1 into the mounting portion 62 from the cartridge insertion port 53A. At this time, since the cartridge 1 is inserted into the mounting portion 62 while the guide plate 26 is being guided by the guide rail 63A, the PCR container 30 of the cartridge 1 is guided to the insertion hole 64A of the mounting portion 62. The operator inserts the cartridge 1 until the fixing claw 25 of the cartridge 1 is hooked on the notch of the fixing portion 63. With this, the cartridge 1 is fixed to a normal position of the mounting portion 62. When the PCR container 30 is not inserted into the insertion hole 64A, and the cartridge 1 is fixed to an abnormal position of the mounting portion 62, the fixing claw 25 of the cartridge 1 is not hooked on the notch of the fixing portion 63. Accordingly, the operator can recognize that the cartridge 1 is at the abnormal position.

As shown in Fig. 9C, when the cartridge 1 is fixed to a normal position of the mounting portion 62, the elution solution plug 47 of the tube 20 faces the heater for elution 65A, the upstream side (high temperature region 36A) of the flow path forming portion 35 of the PCR container 30 faces the high temperature-side heater 65B, and the downstream side (low temperature region 36B) of the flow path forming portion 35 of the PCR container 30 faces the low temperature-side heater 65C. Since the mounting portion 62 and the heaters are disposed in the rotary body 61, even when the rotary body 61 rotates, the positional relationship between the cartridge 1 and the heaters is maintained in this state.

After the cartridge 1 is mounted on the mounting portion 62, as shown in Fig. 9D, the controller 90 controls the rotary body 61 to rotate by 30 degrees such that the rotary body 61 returns to the reference. The controller 90 may detect the state where the cartridge 1 is mounted on the mounting portion 62 using a sensor (not shown). Alternatively, the controller 90 may detect the state by an operator's input operation.

### 2. Nucleic Acid Elution Treatment

### Up-and-Down Motion of Magnets 71

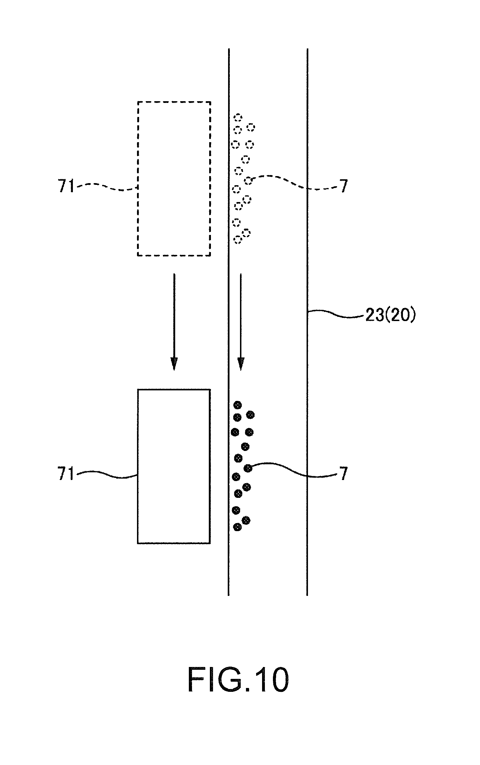

Fig. 10 is a conceptual diagram of the behavior of the magnetic bead 7 when the magnet 71 is moved downward. The magnetic beads 7 in the cartridge 1 are attracted to the magnet 71. For this reason, if the magnet 71 moves outside the cartridge 1, the magnetic beads 7 in the cartridge 1 move along with the magnet 71.

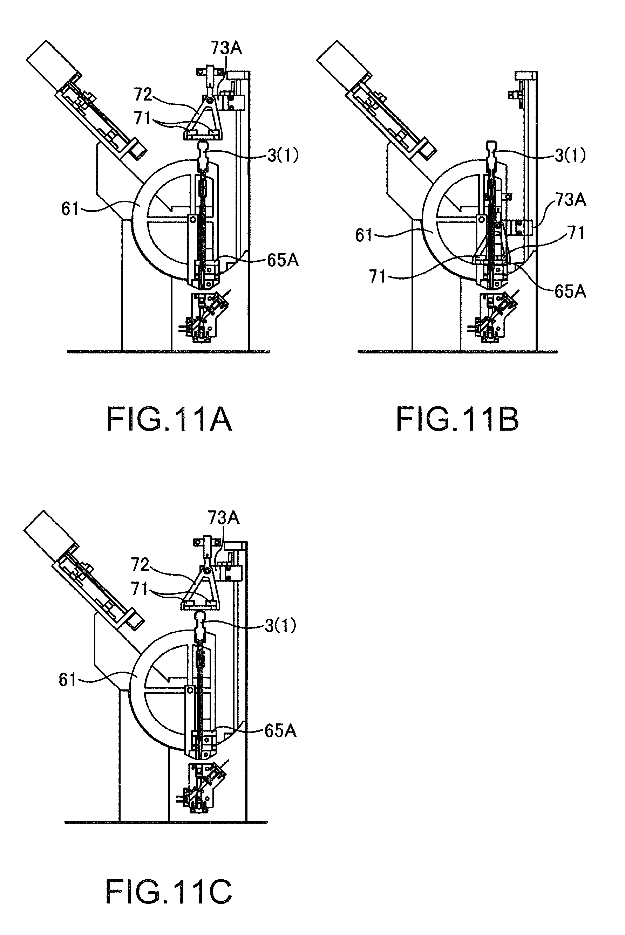

Figs. 11A to 11C are diagrams illustrating a nucleic acid elution process. Fig. 11A is a diagram illustrating the state of the PCR device 50 before the nucleic acid elution process. Fig. 11B is a diagram illustrating the state of the PCR device 50 when the magnets 71 are moved to the elution solution plug 47. Fig. 11C is a diagram illustrating the state of the PCR device 50 when the magnets 71 are drawn up.

As shown in Fig. 11A, in the initial state, the tank 3 becomes the upper side of the cartridge 1, and the longitudinal direction of the cartridge 1 is in parallel with the vertical direction. In this state, as shown in Fig. 2A, the cartridge 1 has the dissolution solution 41 (tank 3) containing the magnetic beads 7, the oil 42 (plunger 10), the washing solution 43 (upstream side of the tube 20), the first oil plug 44 (capillary 23), the washing solution plug 45 (capillary 23), the second oil plug 46 (capillary 23), the elution solution plug 47 (capillary 23), the third oil plug 48 (capillary 23), and the second liquid (PCR container 30) in this order from the upper side of the cartridge.

As shown in Fig. 11A, in the initial state, the carriage 73A is at the uppermost position (retraction position), and the magnets 71 are placed above the cartridge 1. In this state, the controller 90 drives the motor for elevation 73B to slowly move downward the carriage 73A such that the magnets 71 slowly move downward. Since the longitudinal direction of the cartridge 1 is in parallel with the vertical direction, the magnets 71 move along the cartridge 1.

When the magnets 71 move downward, the magnets 71 face the tank 3, and the magnetic beads 7 in the tank 3 are attracted to the magnets 71. The controller 90 moves downward the carriage 73A at such a speed that the magnetic beads 7 can move together with the magnets 71.

When the magnets 71 move from the position (the height of the tank 3) facing the tank 3 to the position (the height of the plunger 10) facing the plunger 10, the magnetic beads 7 pass through the opening on the upstream side of the cylindrical portion 11 of the plunger 10 and then pass through the interface between the dissolution solution 41 in the tank 3 and the oil 42 on the upstream side of the cartridge main body 9. With this, the magnetic beads 7 bound to the nucleic acid are introduced into the cartridge main body 9. When the magnetic beads 7 pass through the interface between the dissolution solution 41 and the oil 42, the dissolution solution 41 is wiped by the oil 42. Therefore, the components of the dissolution solution 41 are not easily brought into the oil 42. With this, it is possible to prevent the components of the dissolution solution 41 from being brought into the washing solution or the elution solution 47.

When the magnets 71 move downward in the state of facing the plunger 10, the magnetic beads 7 pass through the inside of the cylindrical portion 11, escape from the inside and outside of the ribs 13, come out from the opening of the downstream side of the cylindrical portion 11, and are introduced into the upper syringe 21 of the tube 20. In the meantime, the magnetic beads 7 pass through the interface between the oil 42 and the washing solution 43, in the plunger 10. When the magnetic beads 7 are introduced into the washing solution 43, the nucleic acid bound to the magnetic beads 7 is washed with the washing solution 43.

At this stage, the rod-like portion 12 of the plunger 10 is not yet inserted into the lower syringe 22 of the tube 20. Therefore, when the magnets 71 move from the position (the height of the upper syringe 21) facing the upper syringe 21 to the position (the height of the capillary 23) facing the capillary 23, the magnetic beads 7 move to the lower syringe 22 from the upper syringe 21 and then move to the capillary 23 from the lower syringe 22. The first oil plug 44 is on the upstream side of the capillary 23, and when the magnetic beads 7 move to the capillary 23 from the lower syringe 22, the magnetic beads 7 pass through the interface between the washing solution 43 and the oil. At this time, since the washing solution 43 is wiped by the oil, the components of the washing solution 43 are not easily brought into the oil. In this way, it is possible to prevent the components of the washing solution 43 from being mixed into the washing solution plug 45 or the elution solution plug 47.

If the magnets 71 move from the position (the height of the first oil plug 44) facing the first oil plug 44 to the position (the height of the washing solution plug 45) facing the washing solution plug 45, the magnetic beads 7 pass through the interface between the oil and the washing solution. When the magnetic beads 7 are introduced into the washing solution plug 45, the nucleic acid bound to the magnetic beads 7 is washed with the washing solution.

When the magnets 71 move from the position (the height section of the washing solution plug 45) facing the washing solution plug 45 to the position (the height of the second oil plug 46) facing the second oil plug 46, the washing solution and the magnetic beads 7 pass through the interface between the washing solution and the oil. At this time, since the washing solution is wiped by the oil, the components of the washing solution are not easily brought into the oil. With this, it is possible to prevent the components of the washing solution from being mixed into the elution solution plug 47.

When the magnets 71 move from the position (the height of the second oil plug 46) facing the second oil plug 46 to the position (the height of the elution solution plug 47) facing the elution solution plug 47, the magnetic beads 7 pass through the interface between the oil and the elution solution 47.

Before the magnetic beads 7 are introduced into the elution solution plug 47, the controller 90 controls the heater for elution 65A to heat the elution solution plug 47 to about 50°C. If the elution solution 47 is heated before the magnetic beads 7 are introduced into the elution solution 47, the time from when the magnetic beads 7 are introduced into the elution solution 47 until the elution of the nucleic acid is completed can be shortened.

As shown in Fig. 11B, after the magnets 71 move to the position (the height of the elution solution plug 47) facing the elution solution plug 47, the controller 90 stops the motor for elevation 73B to stop the movement of the magnets 71 in the up-down direction and heats the elution solution plug 47 for 30 seconds at 50°C. Therefore, the nucleic acid bound to the magnetic beads 7 is liberated into the solution of the elution solution plug 47, and a reverse transcription reaction proceeds. If the elution solution 47 is heated, the elution of nucleic acid from the magnetic beads 7 and the reverse transcription reaction are accelerated.

After nucleic acid is eluted in the elution solution plug 47, the controller 90 drives the motor for elevation 73B in an opposite direction such that the carriage 73A slowly moves upward to slowly move the magnets 71 upward. The controller 90 moves the carriage 73A upward at such a speed that the magnetic beads 7 can move together with the magnets 71.

When the magnets 71 move upward in the state shown in Fig. 11B, the magnetic beads 7 move to the second oil plug 46 from the elution solution plug 47, whereby the magnetic beads 7 are removed from the elution solution plug 47.

When the magnets 71 slowly move to the position facing the upper syringe 21, the magnetic beads 7 also move to the upper syringe 21 and are positioned above the lower syringe 22. If the magnetic beads 7 are moved to such a position, the magnetic beads 7 are not introduced into the PCR container 30 when the plunger 10 is pushed. Therefore, while the state is changed to the state shown in Fig. 11C from the state described above, the controller 90 may move the carriage 73A upward at such a speed that the magnetic beads 7 are inhibited from following the movement of the magnets 71. In addition, if the magnetic beads 7 are not introduced into the PCR container 30 when the plunger 10 is pushed, the movement speed of the carriage 73A may be increased at the earlier stage.

The storage device of the controller 90 stores information regarding the movement speed of the magnets 71. The controller 90 executes the above operation (operation for moving up and down the magnets 71) according to information.

### Oscillation of Magnets 71

While the magnets 71 are moved vertically, the controller 90 may drive the motor for oscillation 75A such that the pair of magnets 71 with the cartridge 1 sandwiched therebetween oscillates in the front-back direction.

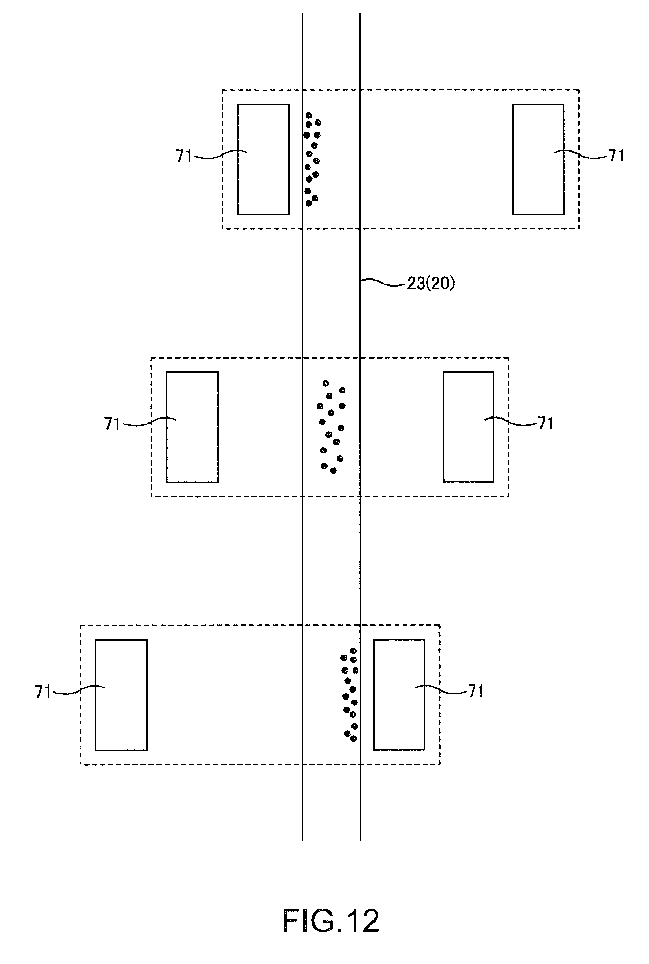

Fig. 12 is a conceptual diagram showing the behavior of the magnetic beads 7 when the magnets 71 oscillate.

While the magnets 71 are moving in the vertical direction, the tube 20 is interposed between the pair of magnets 71 in the front-back direction. Since the pair of magnets 71 is held by the arm 72, the distance between the pair of magnets 71 in the front-back direction is substantially constant. Therefore, one of the pair of magnets 71 approaches the tube 20, and the other magnet is separated from the tube 20.

The magnetic beads 7 are attracted to the magnet 71 close to the magnetic beads. Therefore, when one of the pair of magnets 71 approaches the tube 20, the magnetic beads 7 are attracted to the magnet 71. Thereafter, when the magnet 71 is separated from the tube 20, and then the other magnet 71 approaches the tube 20, the magnetic beads 7 are attracted to the magnet 71. In this way, the magnetic beads 7 move in the front-back direction. When the pair of magnets 71 oscillates in the front-back direction, the magnetic beads 7 reciprocate in the front-back direction.

When the magnetic beads 7 reciprocate in the front-back direction, the magnetic beads 7 easily come into contact with a liquid. Particularly, since the liquid in the capillary 23 hardly exhibits fluidity, when it is desired to make the liquid in the capillary 23 become as close to the magnetic beads 7 as possible, it is effective to cause the magnetic beads 7 to reciprocate in the front-back direction.

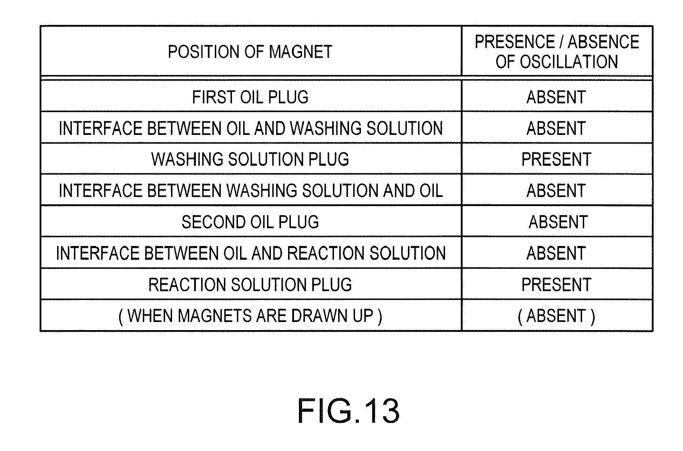

Fig. 13 is a table showing the presence/absence of oscillation of the magnets 71.

When the magnetic beads 7 move the oil plug (the first oil plug 44 or the second oil plug 46) downward, the controller 90 stops the motor for oscillation such that the magnets 71 do not oscillate. At this time, the controller 90 moves the magnets 71 downward in a state where one of the pair of magnets 71 is brought close to the tube 20. This is because the magnetic beads 7 more easily follow the movement of the magnets 71 compared to the case where the magnets 71 are separated from the tube 20 by the same distance.

When the magnetic beads 7 move the washing solution plug 45 downward, the controller 90 drives the motor for oscillation to oscillate the magnets 71 in the front-back direction. With this, the magnetic beads 7 move downward while oscillating in the front-back direction inside the washing solution plug 45. Therefore, the washing effect of the magnetic beads 7 can be enhanced. Since the washing effect is enhanced, the amount of the washing solution plug 45 can be reduced, and the cartridge 1 can be reduced in size.

When the magnetic beads 7 pass through the interface between the washing solution and oil (second oil plug 46), the controller 90 stops the motor for oscillation such that the magnets 71 do not oscillate. With this, since the magnetic beads 7 do not oscillate when passing through the interface, the components of the washing solution are not easily brought into the oil. The controller 90 moves the magnets 71 downward in a state where one of the pair of magnets 71 is brought close to the tube 20. Therefore, the magnetic beads 7 close to the magnet 71 are attracted to the magnet and are aggregated, and the washing solution adhered to the magnetic beads 7 is squeezed out. Therefore, the components of the washing solution are not easily brought into the oil.

When the magnetic beads 7 are in the elution solution plug 47, the controller 90 drives the motor for oscillation such that the magnets 71 oscillate in the front-back direction. With this, since the magnetic beads 7 oscillate in the front-back direction inside the elution solution plug 47, the elution effect of the nucleic acid bound to the magnetic beads 7 can be enhanced. Since the elution effect is enhanced, the time from when the magnetic beads 7 are introduced into the elution solution 47 to when the elution of the nucleic acid is completed can be shortened.

When the nucleic acid is eluted in the elution solution plug 47, and then the magnets 71 are moved upward to draw up the magnetic beads 7, the controller 90 stops the motor for oscillation such that the magnets 71 do not oscillate. At this time, the controller 90 moves the magnets 71 downward in a state where one of the pair of magnets 71 is brought close to the tube 20. With this, the magnetic beads 7 easily follow the movement of the magnets 71, and the movement speed of the magnets 71 can be increased.

The storage device of the controller 90 stores information regarding the position of each plug of the capillary 23 and information regarding oscillation as shown in Fig. 13. The controller 90 executes the above operation (operation for oscillating the magnets 71) according to information.

### 3. Droplet Forming Process

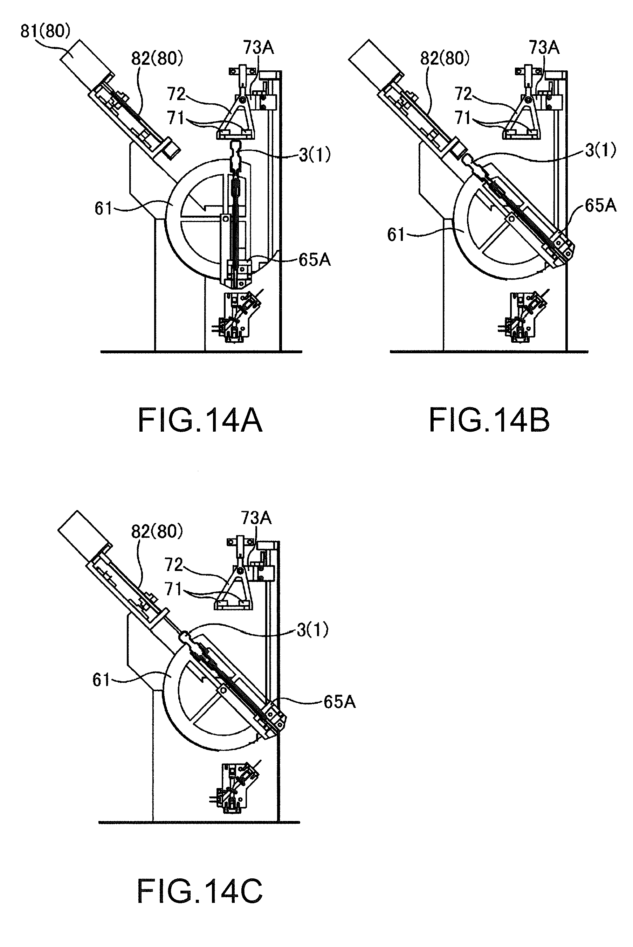

Figs. 14A to 14C are diagrams illustrating a droplet formation process. Fig. 14A is a diagram illustrating the state of the PCR device 50 when the magnets 71 are drawn up. Fig. 14B is a diagram illustrating the state where the rotary body 61 is rotated by 45 degrees. Fig. 14C is a diagram illustrating the state where the rod 82 of the pushing mechanism 80 pushes the plunger 10.

As shown in Fig. 14A, when the carriage 73A is at the retraction position, the elevating mechanism 73 does not come into contact with the cartridge 1 even when the cartridge 1 rotates. After this state is placed, the controller 90 rotates the rotary body 61 by 45 degrees.

As shown in Fig. 14B, when the rotary body 61 rotates by 45 degrees, the longitudinal direction of the cartridge 1 is in parallel with the movement direction of the rod 82 of the pushing mechanism 80. The controller 90 drives the motor for a plunger 81 to move the rod 82. When the rod 82 comes into contact with the mounting board 11A of the plunger 10 of the cartridge 1 and then moves further, the plunger 10 is pushed into the tube 20. The controller 90 moves the rod 82 until the state shown in Fig. 14C is placed and pushes the plunger 10 until the mounting board 11A of the plunger 10 comes into contact with the upper rim of the tube 20.

If the plunger 10 is pushed into the tube 20, the seal 12A of the rod-like portion 12 of the plunger 10 fits to the lower syringe 22 of the tube 20 (see Fig. 2B). Thereafter, if the plunger 10 is further pushed, the seal 12A slides inside the lower syringe 22. With this, the liquid (the third oil plug 48, the elution solution plug 47, and the like) on the downstream side of the tube 20 is pushed out to the flow path forming portion 35 of the PCR container 30 by an amount corresponding to the volume in which the seal 12A slides inside the lower syringe 22.

First, the third oil plug 48 of the tube 20 flows into the flow path forming portion 35. Since the flow path forming portion 35 is filled with oil, the inflow oil flows into the oil accommodating space 32A from the flow path forming portion 35, whereby the oil interface of the oil accommodating space 32A ascends. At this time, due to pressure loss of the lower seal portion 34B, the pressure of the liquid of the flow path forming portion 35 becomes higher than the outside pressure (the pressure of the oil accommodating space 32A). After the third oil plug 48 is pushed out from the tube 20, the elution solution plug 47 flows into the flow path forming portion 35 from the tube 20. Since the inner diameter of the flow path forming portion 35 is greater than the inner diameter of the capillary 23, the elution solution 47, which is made in the form of a plug in the tube 20, is formed into a droplet in the oil of the flow path forming portion 35.

The seal 12A slides inside the lower syringe 22, and the amount of the liquid in the tube 20 which is pushed out from the downstream side is greater than the total volume of the elution solution plug 47 and the third oil plug 48 in the tube 20. Therefore, after the elution solution plug 47 is pushed out from the tube 20, a portion of the second oil plug 46 is also pushed out to the flow path forming portion 35. With this, the elution solution 47 does not remain in the tube 20, and the entire elution solution plug 47 is formed into a droplet. A portion of the second oil plug 46 may be pushed out from the downstream side of the tube 20, whereby the elution solution 47 in the form of a droplet is not easily adsorbed onto the opening of the capillary 23 and easily leaves the tube 20.

The capillary 23 is designed such that the inner diameter of the terminal thereof (the diameter of the opening of the capillary 23) is comparatively small. Therefore, the elution solution 47, which has been formed in a droplet in the PCR container 30, is not easily adsorbed onto the opening of the capillary 23. The specific gravity of the elution solution 47 is greater than that of the second liquid of the PCR container 30. For this reason, the elution solution 47 in the form of a droplet leaves the terminal of the capillary 23, passes through the flow path forming portion 35 as a flow path, and precipitates toward the bottom 35A. Then, the elution solution 47 in the form of a droplet reaches the bottom 35A of the PCR container 30, stops to precipitate, comes into contact with the freeze-dried nucleic acid amplification reaction reagent, and dissolves the nucleic acid amplification reaction reagent. However, at this stage, since the flow path of the flow path forming portion 35 is inclined by 45 degrees, the elution solution 47 in the form of a droplet is easily adhered to the inner wall of the flow path forming portion 35. Therefore, the flow path of the flow path forming portion 35 should be returned to the vertical direction.

After the plunger 10 is pushed and the elution solution 47 in the form of a droplet is formed, the controller 90 drives the motor for a plunger 81 in an opposite direction such that the rod 82 returns to the original position. In this state, even if the cartridge 1 rotates, the rod 82 of the pushing mechanism 80 does not come into contact with the cartridge 1. After this state is placed, the controller 90 returns the rotary body 61 to the reference position. When the rotary body 61 returns to the reference position, the flow path of the flow path forming portion 35 is in the vertical direction. Therefore, the elution solution 47 in the form of a droplet does not easily adhere to the inner wall of the flow path forming portion 35.

In this way, when a plug containing nucleic acid is ejected to the PCR container 30, one or more oil plugs in the capillary 23 are ejected to the PCR container 30. As described above, the first additive is contained in at least one of these oil plugs. The first liquid in the capillary 23 contains an additive of a higher concentration than the second liquid in the PCR container 30. Therefore, the first additive contained in the first liquid in the capillary 23 is diluted in the PCR container 30. As a result, the concentration of the additive in the PCR container 30 is equal to or greater than 1% (v/v) and equal to or less than 50% (v/v).

### 4. Thermal Cycle Treatment

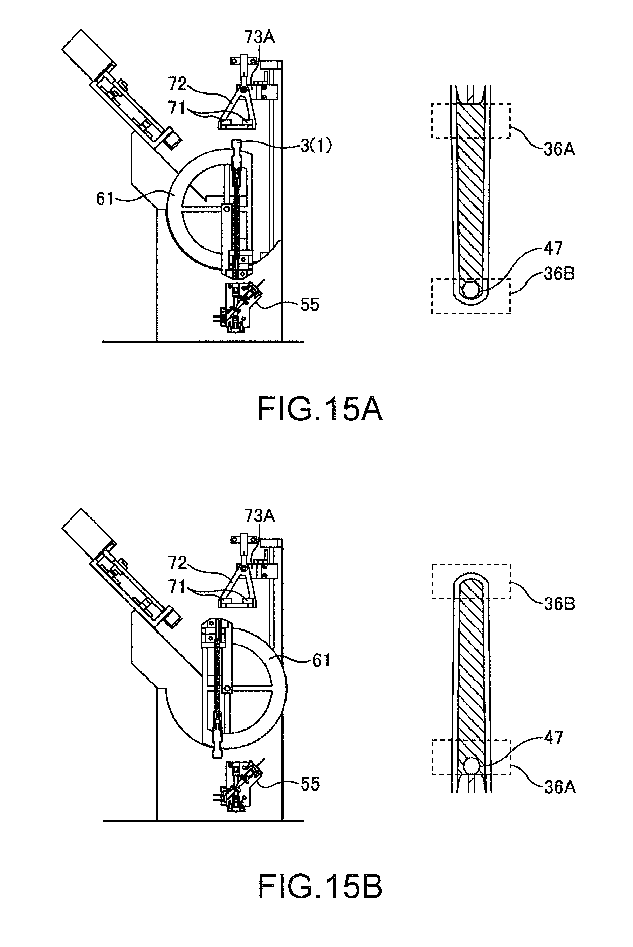

Figs. 15A and 15B are diagrams illustrating a thermal cycle process. Fig. 15A is a diagram illustrating the state where the elution solution 47 is subjected to a temperature process on a low temperature side. Fig. 15B is a diagram illustrating the state where the elution solution 47 is subjected to a temperature process on a high temperature side. The left side of each drawing represents the state of the PCR device 50, and the right side of each drawing represents the internal state of the flow path forming portion 35 of the PCR container 30.

When the cartridge 1 is fixed to a normal position in the mounting portion 62, the upstream side (high temperature region 36A) of the flow path forming portion 35 of the PCR container 30 faces the high temperature-side heater 65B, and the downstream side (low temperature region 36B) of the flow path forming portion 35 of the PCR container 30 faces the low temperature-side heater 65C. During the thermal cycle process, the controller 90 controls the high temperature-side heater 65B in the rotary body 61 such that the liquid of the high temperature region 36A on the upstream side of the flow path forming portion 35 of the PCR container 30 is heated to about 90°C to 100°C. The controller 90 controls the low temperature-side heater 65C in the rotary body 61 such that the liquid of the low temperature region 36B on the downstream side of the flow path forming portion 35 is heated to about 50°C to 75°C. With this, during the thermal cycle process, a temperature gradient is formed in the liquid in the flow path forming portion 35 of the PCR container 30. Since the mounting portion 62 and the heaters are provided in the rotary body 61, the positional relationship between the cartridge 1 and the heaters is maintained in this state even when the rotary body 61 rotates. When RT-PCR is performed in PCR container 30, before the thermal cycle process, a reverse transcription reaction may be performed by processing the elution solution 47, in which the nucleic acid amplification reaction reagent is dissolved, at about 42 to 55°C by the low temperature-side heater 65C.

During the thermal cycle process, the liquid in the PCR container 30 is heated. If air bubbles are formed in the liquid of the PCR container 30 due to heating of the liquid, the temperature of the liquid in the flow path forming portion 35 may become uneven, or movement (precipitation) of the elution solution 47 in the form of a droplet in the flow path forming portion 35 may be hindered. However, in this embodiment, due to pressure loss of the lower seal portion 34B, the pressure of the liquid of the flow path forming portion 35 is higher than the outside pressure. Therefore, air bubbles are not easily formed in the liquid of the PCR container 30.

As shown in Fig. 15A, when the rotary body 61 is at the reference position, the low temperature-side heater 65C is positioned below the high temperature-side heater 65B, and the bottom 35A of the PCR container 30 of the cartridge 1 becomes the lower side. Since the specific gravity of the elution solution 47 in the form of a droplet is greater than that of the second liquid, the elution solution 47 in the form of a droplet precipitates inside the flow path forming portion 35. If precipitating inside the flow path forming portion 35, the elution solution 47 in the form of a droplet reaches the bottom 35A of the PCR container 30. The elution solution 47 stops to precipitate at the bottom 35A and stays in the low temperature region 36B. With this, the elution solution 47 in the form of a droplet moves to the low temperature region 36B. If the controller 90 maintains the state shown in Fig. 15A for a predetermined time and heats the elution solution 47 in the form of a droplet to about 50°C to 75°C in the low temperature region 36B, a temperature process on the low temperature side is performed, and an extension reaction of the polymerase reaction occurs.

If the controller 90 drives the motor for rotation 66 in the state shown in Fig. 15A to rotate the rotary body 61 by 180 degrees, the state shown in Fig. 15B is placed. When the rotary body 61 rotates by 180 degrees from the reference position, the cartridge 1 becomes upside down, and the high temperature-side heater 65B as well as the low temperature-side heater 65C also become upside down. That is, the high temperature-side heater 65B is positioned below the low temperature-side heater 65C, and the bottom 35A of the PCR container 30 of the cartridge 1 becomes the upper side. After precipitating inside the flow path forming portion 35, the elution solution 47 in the form of a droplet reaches the terminal of the tube 20. Then, the elution solution 47 stops to precipitate at the terminal and stays in the high temperature region 36A. With this, the elution solution 47 in the form of a droplet moves to the high temperature region 36A. If the controller 90 maintains the state of Fig. 15B for a predetermined time and heats the elution solution 47 in the form of a droplet to about 90°C to 100°C in the high temperature region 36A, a temperature process on the high temperature side is performed, and the nucleic acid is denatured.

If the controller 90 drives the motor for rotation 66 in the state of Fig. 15B to rotate the rotary body 61 by -180°, the state of Fig. 15A is returned. In this state, if precipitating inside the flow path forming portion 35, the elution solution 47 in the form of a droplet moves to the low temperature region 36B and is heated again to about 50°C to 75°C in the low temperature region 36B, and a temperature process on the low temperature side is performed. Since the capillary 23 is designed such that the inner diameter of the terminal thereof, that is, the diameter of the opening of the capillary 23 is comparatively small, the elution solution 47 is not easily adsorbed onto the opening of the capillary 23. Therefore, if the rotary body 61 rotates by -180° in the state of Fig. 15B, the elution solution 47 in the form of a droplet leaves the tube 20 and precipitates toward the bottom 35A of the PCR container 30 without being adsorbed onto the opening of the capillary 23.

The controller 90 drives the motor for rotation 66 such that the rotation position of the rotary body 61 is placed in the state of Fig. 15A and the state of Fig. 15B. The controller 90 repeats this operation for a predetermined number of cycles. With this, the PCR device 50 can perform the thermal cycle process of PCR on the elution solution 47.

The storage device of the controller 90 stores thermal cycle information regarding the temperature of the high temperature-side heater 65B, the temperature of the low temperature-side heater 65C, the time during which the PCR device 50 is held in the state of Fig. 15A, the time during which the PCR device 50 is held in the state of Fig. 15B, and the number of cycles (the number of repetitions of the state of Fig. 15A and the state of Fig. 15B). The controller 90 executes the above process according to the thermal cycle information.

### 5. Fluorescence Measurement in Real Time PCR

As shown in Fig. 15A, when the rotary body 61 is at the reference position, the fluorescence measuring instrument 55 faces the bottom 35A of the PCR container 30 of the cartridge 1. For this reason, when measuring fluorescence of the elution solution 47, the controller 90 causes the fluorescence measuring instrument 55 to measure fluorescence intensity of the elution solution 47 at the bottom 35A of the PCR container 30 from the lower opening of the insertion hole 64A of the low temperature-side heater 65C in a state where the rotary body 61 is at the reference position.

Immediately after the rotary body 61 rotates by 180 degrees and is at the reference position, the elution solution 47 in the form of a droplet precipitates inside the flow path forming portion 35 of the PCR container 30, and the elution solution 47 in the form of a droplet may not reach the bottom 35A of the PCR container 30. For this reason, it is desirable that the controller 90 measures fluorescence intensity when a predetermined time elapses after the rotation position of the rotary body 61 is placed in the state of Fig. 15A (immediately before the rotary body 61 is rotated from the state of Fig. 15A). Alternatively, the controller 90 may cause the fluorescence measuring instrument 55 to measure fluorescence intensity for a predetermined time after the rotary body 61 is at the reference position and may store a time history of fluorescence intensity.

### Other Aspects

The above embodiments are for ease of understanding of the invention, and the invention is not limited to these embodiments. Needless to say, the invention can be modified or improved without departing from the spirit of the invention, and the invention also includes the equivalents thereof.

### Regarding PCR Device

The PCR device 50 changes the posture of the cartridge 1 by a predetermined number of cycles to perform a thermal cycle process and moves the PCR container 30 upside down. However, the number of changes of the posture of the cartridge 1 is not limited to multiple times and may be once.

In the above-described PCR device, the rotary shaft of the rotary body is positioned closer to the tube than to the PCR container. However, the position of the rotary shaft of the rotary body is not limited thereto as long as the droplet can move inside the PCR container when the posture of the cartridge is changed due to the rotation of the rotary body.

In the above-described PCR device, the fixing portion 63, in which a notch is formed, and the insertion hole 64A constitutes the mounting portion of the cartridge. However, the mounting portion is not limited to this configuration as long as the cartridge can be mounted on the rotary body. The mounting portion may have a structure in which the cartridge is fixed to the rotary body by only fixing the tube side, or may have a structure in which the cartridge is fixed to the rotary body by only fixing the PCR container side. However, the mounting portion should have a structure in which the cartridge is stably fixed to the rotary body even if the posture of the cartridge is changed due to the rotation of the rotary body.

The above-described PCR device 50 has the high temperature-side heater 65B and the low temperature-side heater 65C as the heaters for PCR. However, the device is not limited to this configuration as long as a temperature gradient can be formed inside the PCR container as a container for a nucleic acid amplification reaction. For example, the heater may be provided only on a high temperature side. Alternatively, a heater may be provided on a high temperature side, and a cooler may be provided on a low temperature side.

In the above-described PCR device 50, the heaters for PCR (the high temperature-side heater 65B and the low temperature-side heater 65C) are provided in the rotary body. However, the heaters for PCR may be provided in the outside of the rotary body as long as a temperature gradient can be formed inside the PCR container as a container for a nucleic acid amplification reaction. For example, a first heater for PCR, which faces the PCR container when the rotary body 61 is at the reference position as shown in Fig. 15A, and a second heater for PCR, which faces the PCR container when the rotary body rotates by 180 degrees as shown in Fig. 15B, may be provided in the outside of the rotary body of the PCR device. Even in this structure, a temperature gradient can be formed inside the PCR container as a container for a nucleic acid amplification reaction. However, it is desirable that the heaters for PCR are provided in the rotary body since the positional relationship between the PCR container of the cartridge and the heaters can be maintained regardless of the rotation position of the rotary body.

The PCR device 50 may only have the heaters for PCR (for example, the high temperature-side heater 65B and the low temperature-side heater 65C) without the heater for elution 65A. However, it is desirable that the PCR device 50 has the heater for elution 65A since the liberation of nucleic acid from magnetic beads is accelerated.

The PCR device 50 may not have the magnet moving mechanism which moves the magnets along the tube. In this case, for example, the operator may grab and move the magnets along the tube. However, it is desirable that the PCR device 50 has the magnet moving mechanism since the movement speed and the like of the magnetic beads as nucleic acid-binding solid-phase carriers may be changed by the operator.

The magnet moving mechanism 70 of the PCR device 50 may not have the oscillating mechanism 75. In this case, although the magnets cannot oscillate, the magnets can be moved along the tube. Therefore, the magnetic beads bound to nucleic acid can be moved to the plug containing the elution solution.

The PCR device 50 may not have the pushing mechanism 80 or may have alternative pushing means. In this case, for example, the plunger or the tank may become pushing means. That is, the operator may push the plunger of the cartridge by hand. When the plunger is not provided in the cartridge, the operator may deform a tank made of a deformable material described above, whereby the inside of the tank may be pressed to push out the liquid from the tube to the PCR container.

### Examples

### Experimental Example 1

### PCR Using Above PCR Device

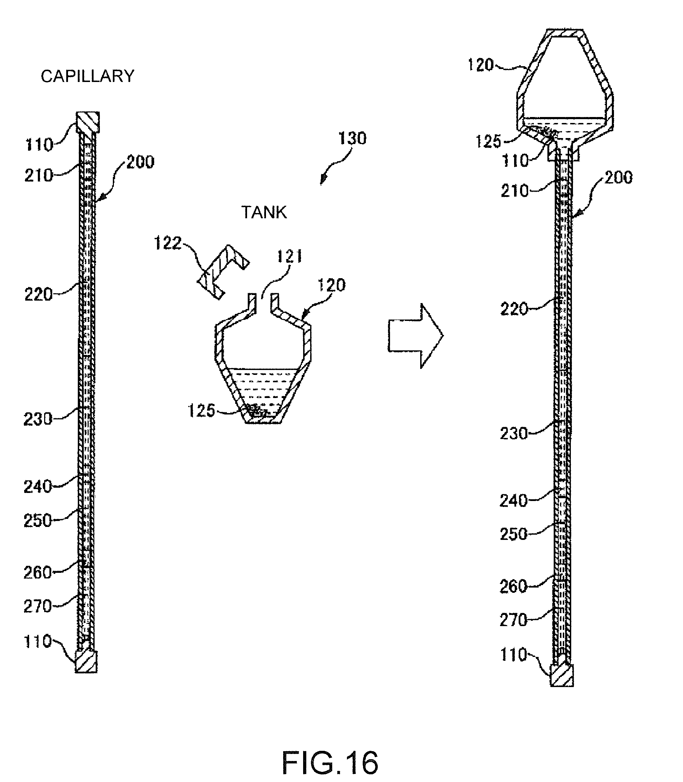

In this experimental example, as shown in Fig. 16, a configuration in which a first plug 210 to a seventh plug 270 are contained inside a tube 200 of the above-described kit for nucleic acid extraction was used.

First, 375 µL of a dissolution solution and 1 µL of a magnetic bead dispersion were put in a polyethylene container 130 with a capacity of 3 mL. As the dissolution solution, an aqueous solution containing 76% by mass of guanidine hydrochloride, 1.7% by mass of ethylenediaminetetraacetic acid disodium salt dehydrate, and 10% by mass of polyoxyethylene sorbitan monolaurate (manufactured by TOYOBO Co., Ltd., MagExtractor-Genome, NPK-1) was used. As the magnetic bead stock solution, a stock solution containing 50% by volume of magnetic silica particles and 20% by mass of lithium chloride was used.

50 µL of blood collected from a human being was put in a container 130 from an opening 121 using a pipette, the container 130 was covered with a lid 122, and the container was manually shaken for 30 seconds to stir the content in the container. Thereafter, the lid 122 of the container 130 was removed, and the container was connected to the tube 200. Both ends of the tube 200 were sealed with a stopper 110. The stopper 110 on the first plug 210 side was removed, and the container 130 was connected to the tube 200.

Silicone oil was used as the first plug 210, the third plug 230, the seventh plug 270, and the fifth plug 250. As a first washing solution of the second plug 220, an aqueous solution containing 76% by mass of guanidine hydrochloride was used. As a second washing solution of the fourth plug 240, a Tris-HCl buffer solution (solute concentration of 5 mM) with pH of 8.0 was used. As the elution solution of the sixth plug 260, sterile water was used.

Subsequently, a permanent magnet 410 was moved to introduce magnetic beads 125 in the container 130 into the tube 200. Then, the magnetic beads 125 were moved to the sixth plug 260. The time during which the magnetic beads 125 stayed in each plug in the tube 200 was as follows; the first, third, and seventh plugs: 3 seconds, the second plug: 20 seconds, the fourth plug: 20 seconds, and the sixth plug: 30 seconds. In the second plug 220 and the fourth plug 240, an operation for oscillating the magnetic beads was not performed. The volume of the second plug 220, the fourth plug 240, and the sixth plug 260 were respectively 25 µL, 25 µL, and 1 µL.

Next, the stopper 110 on the seventh plug side of the tube was removed, and the container 120 was manually deformed such that the seventh plug 270 and the sixth plug 260 were ejected to the PCR reaction container. This operation was performed after the magnetic beads were moved using the permanent magnet to cause the magnetic beads to be retracted to the second plug 220.

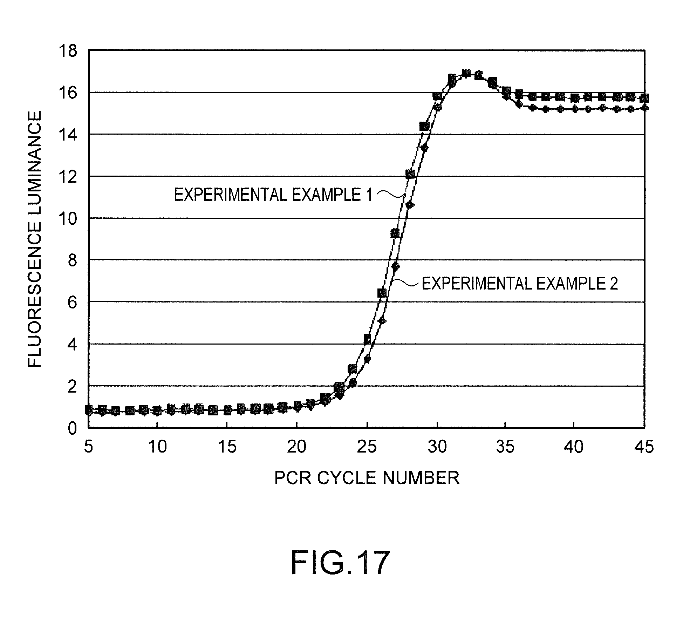

Then, 19 µL of a PCR reaction reagent was added to the extract obtained as above to perform real time PCR according to the usual method. The PCR reaction reagents were composed of 4 µL of LightCycler 480 Genotyping Master (manufactured by Roche Diagnostics, 4 707 524), 0.4 µL of SYBR Green I (manufactured by Life Technologies Corporation, S7563) diluted with sterile water by 1,000-fold, 0.06 µL of 100 µM primers (F/R) for β actin detection, and 14.48 µL of sterile water. Fig. 17 shows a PCR amplification curve of Experimental Example 1. In Fig. 17, the ordinate indicates fluorescence intensity, and the abscissa indicates the number of PCR cycles.

### Comparative Example

In this comparative example, nucleic acid was extracted by a general nucleic acid extraction method.

First, 375 µL of a dissolution solution and 20 µL of a magnetic bead dispersion were put in a polyethylene container (Eppendorf tube) with a capacity of 1.5 µL. The composition of the dissolution solution and the magnetic bead dispersion was the same as in the above experiment example.

Next, 50 µL of blood collected from a human being was put in the container from the opening thereof using a pipette, and the container was covered with a lid. Then, the content of the container was stirred for 10 minutes using a vortex mixer, and a B/F separation operation was performed using a magnetic stand and a pipette. In this state, the magnetic beads and a small amount of dissolution solution remained in the container.

Next, 450 µL of the first washing solution having the same composition as when the above PCR device is used was put in the container, the container was covered with a lid, the content in the container was stirred for 5 seconds with a vortex mixer, and then the first washing solution was removed using a magnetic stand and a pipette. This operation was repeated twice. In this state, the magnetic beads and a small amount of first washing solution remained in the container.

Next, 450 µL of the second washing solution having the same composition as when the above PCR device is used was put in the container, the container was covered with a lid, the content in the container was stirred for 5 seconds with a vortex mixer, and the second washing solution was removed using a magnetic stand and a pipette. This operation was repeated twice. In this state, the magnetic beads and a small amount of second washing solution remained in the container.

Then, 50 µL of sterile water (elution solution) was added to the container, the container was covered with a lid, the content in the container was stirred with a vortex mixer for 10 minutes, and the supernatant liquid was collected by operating a magnetic stand and a pipette. The supernatant liquid contained the target nucleic acid.

Then, 1 µL of the extract obtained as above was dispensed, and 19 µL of a PCR reaction reagent was added thereto to perform real time PCR according to the usual method. The PCR reaction reagent was composed of 4 µL of LightCycler 480 Genotyping Master (manufactured by Roche Diagnostics, 4 707 524), 0.4 µL of SYBR Green I (manufactured by Life Technologies Corporation, S7563) diluted with sterile water by 1,000-fold, 0.06 µL of 100 µM primers (F/R) for β actin detection, and 14.48 µL of sterile water. Fig. 17 shows a PCR amplification curve obtained at this time.

### Experimental Results

From this experiment example, the following can be understood.

(1) By comparison in terms of the time required for the nucleic acid extraction process which is a pre-process of PCR, it was found that the time from when the sample is put in the container until the target nucleic acid is introduced into the PCR reaction container was about 2 minutes when the above PCR device is used and about 30 minutes in Comparative example. This shows that the time required for extracting the nucleic acid is significantly shortened in the nucleic acid extraction method when the above PCR device is used, compared to the nucleic acid extraction method of Comparative example.

(2) The amount of each washing solution when the above PCR device is used was about one eighteenth of that of Comparative example. The amount of the elution solution when the above PCR device is used was about one fiftieth of that of Comparative example. Therefore, it is understood that, when the above PCR device is used, an extremely small amount of washing solution and elution solution is enough.

(3) By comparison in terms of the concentration of the target nucleic acid in the elution solution based on the amount of the dissolution solution and the elution solution, ideally, the concentration of the target nucleic acid when the above PCR device is used will be 50 times higher than that in Comparative Example. However, in the present experimental example, the amount of nucleic acid contained in the blood sample was large and exceeded the possible adsorption amount of 1 µL of magnetic beads, and the entire nucleic acid contained in the blood sample could not be collected. Accordingly, the concentration of nucleic acid when the PCR device is used was not 50 times higher than that in Comparative Example. When a sample having the amount of nucleic acid contained, which is small and does not exceed the possible adsorption amount of at least 1 µL of magnetic beads, is used, the concentration of nucleic acid when the above PCR device is used is 50 times higher than that in Comparative Example.

(4) As shown in the graph of Fig. 17, even in the whole blood sample containing a large amount of nucleic acid, the nucleic acid amplification rate is higher when the above PCR device is used than in Comparative Example by about 0.6 cycles. That is, the concentration of the target nucleic acid is higher in the PCR reaction solution used when the above PCR device is used than in the PCR reaction solution used in Comparative example. In other words, the concentration of the target nucleic acid in the elution solution is higher when the above PCR device is used than in Comparative Example.

### Experimental Example 2

### Preservation Stability of Freeze-dried Reagent

In this example, comparison of preservation stability was performed when a nucleic acid amplification reagent is in the form of a solution and when a nucleic acid amplification reagent is freeze-dried.

First, 10 µL of an adjusted nucleic acid amplification solution was put in an Eppendorf PCR tube (nucleic acid amplification reaction container) with a capacity of 200 µL, one sample was in the form of a solution, and the other sample was applied with trehalose (100 mg/mL) and freeze-dried. The nucleic acid amplification solution is as follows.
0.8 µM Primer F: GAC CAA TCC TGT CAC CTC TGA C (SEQ ID NO: 1)
0.8 µM Primer R: AGG GCA TTT TGG ACA AAG CGT CTA (SEQ ID NO: 2)
0.2 µM probe
TaqMan probe: FAM- TGC AGT CCT CGC TCA CTG GGC ACG -TAMRA (SEQ ID NO: 3)
1xSuperScript III RT/Platimun Taq Mix
1xPCR Master Mix

The freeze-dried nucleic acid amplification reagent was laminated with a second liquid and preserved at room temperature (25°C). The nucleic acid amplification reagent was dissolved with 10 µL of an RNA template after several days to 1 month, and an RT-PCR reaction was examined. The conditions for RT-PCR are as follows.
Reverse transcription 50°C 60 seconds
Enzyme inactivation 95°C 10 seconds
Denaturation 95°C 5 seconds
Annealing or extension 57°C 20 seconds
(50 cycles)

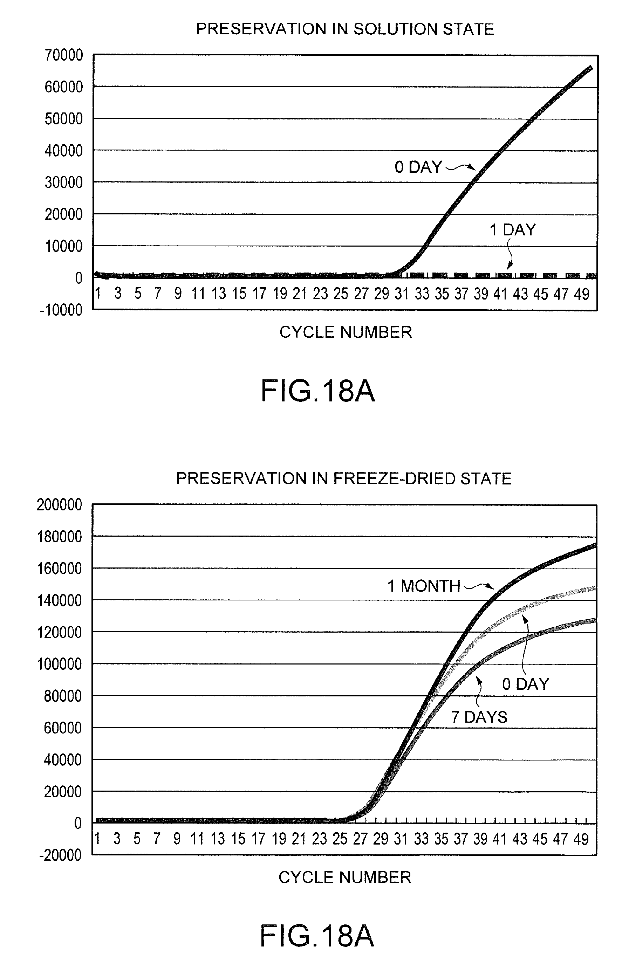

As shown in Fig. 18A, the nucleic acid amplification reagent cannot be preserved even for one day in the form of a solution. Meanwhile, as shown in Fig. 18B, the nucleic acid amplification reagent can be preserved for 1 month or more in a freeze-dried state.

In this way, if the nucleic acid amplification reagent is in a freeze-dried state, even in a sample after preservation for 1 month or more at room temperature, a reaction occurs just like immediately after preparation. Even if a freeze-dried product is covered with the second liquid, a reaction occurs without being hindered by the second liquid.

### Experimental Example 3

### Droplet Adherence Effect by Surfactant in PCR Solution

In this experimental example, surfactant solutions having various concentrations were created using the concentration of a surfactant contained in a commercially available enzyme (DNA polymerase) as a reference, and the adherence of the solutions to the tube was observed.

First, a polypropylene tube for an elevation type PCR device was filled with a second liquid, and 1.6 µL of a surfactant solution was added to the second liquid to create a droplet of the surfactant solution and was kept standstill for 5 minutes. Thereafter, the tube was inclined to separate the surfactant solution from the bottom of the tube, and the tube was erected again. Then, the tube was reversed one minute after the surfactant solution dropped to the bottom, and the adherence of the surfactant solution to the tube bottom was evaluated. A case where a surfactant solution was dropped readily was marked with O, and a case where there was any sign of adherence, for example, a surfactant solution was dropped by shaking or was not dropped even by shaking was marked with X. As oil, KF-96L-2cs which is high-purity methyl silicone oil was used.

As a result, when the surfactant becomes a given concentration or more, the adherence to the bottom of the tube occurred. In Table 1, for the used tube, a maximum concentration in which the adherence to the bottom of the tube does not occur was described. That is, when the concentration of the surfactant is equal to or less than the concentration described in Table 1, the adherence of the reaction solution to the inner wall was not observed. Meanwhile, when the concentration of the surfactant is higher than the concentration described in Table 1, the adherence of the reaction solution to the inner wall was observed.

**Table 1**

| | |
|---|---|
| NP40 | 0.002 |
| Triton-X100 | 0.01 |
| Tween20 | 0.1 |

The unit is % (v/v)

In this way, if a surfactant of a given concentration or more is contained in the reaction solution, this results in a phenomenon in which the reaction solution is adhered to the inner wall of the tube, and even if the tube is reversed, the reaction solution is not dropped.

### Experimental Example 4

### Droplet Adherence Prevention Effect by Additive in Second Liquid

In this experimental example, an additive shown in Table 2 was added and mixed into 2 cs of high-purity methyl silicone oil at 5% (v/v) and was used as a second liquid. In this way, the effect of the additive was confirmed. The reaction solution was prepared as follows.

| | |
|---|---|
| Takara Ex Taq HS (x50) | 0.4 |
| 10x buffer | 1.0 |
| dNTP (10 mM) | 0.2 |
| InfA forward primer (5 uM) | 1.0 |
| InfA reverse primer (5 uM) | 0.3 |
| InfA probe (2 uM) | 0.25 |
| plasmid | 1.0 |
| DW | up to 10.0 (the unit is µL) |

Since 0.5% of NP40 and 0.5% of Tween20 are contained commercially available Takara Ex Taq HS, 0.02% or NP40 and 0.02% of Tween20 are contained in the reaction solution.

As in Experimental Example 4, the adherence of the reaction solution to the tube was observed using the second liquid and the reaction solution. The adherence to the tube was evaluated as in (1).

**Table 2**

| Additive | Content of Additive | Evaluation |
|---|---|---|
| X-22-160AS | carbinol-modified silicone resin | O |
| X-22-3701E | carboxyl-modified silicone resin | O |
| KF-857 | amino-modified silicone resin | O |
| KF-859 | amino-modified silicone resin | O |
| KF-862 | amino-modified silicone resin | O |
| KF-867 | amino-modified silicone resin | O |
| KF-6017 | polyether-modified silicone resin | O |
| KF-8005 | amino-modified silicone resin | O |
| SR1000 | silanol-modified silicone resin | O |
| SS4230 | silanol-modified silicone resin | O |
| SS4267 | silanol-modified silicone resin | O |
| TSF4703 | amino-modified silicone resin | O |
| TSF4708 | amino-modified silicone resin | O |
| YR3370 | silanol-modified silicone resin | O |
| XF42-C5196 | amino-modified silicone resin | O |
| XF42-C5197 | amino-modified silicone resin | O |
| XS66-C1191 | fluoro-modified silicone resin | O |
| None | - | X |

In this way, if the additive shown in Table 2 is added at about 5% (v/v), the adherence of the reaction solution to the tube can be prevented.

### Experimental Example 5

### Destabilization of Nucleic Acid Amplification Reagent by Additive

As in Experimental Example 2, a nucleic acid amplification reagent was freeze-dried in a polypropylene tube for an elevation type PCR device. The tube in which the freeze-dried nucleic acid amplification reagent was put was filled with a second liquid added with an additive SS4230 of 1%, 5%, 10%, and 20%, and the tube was sealed and preserved at 30°C. Then, PCR was performed by an elevation type PCR device described in JP-A-2009-136250 every month. When Ct is delayed by two cycles or more or when luminance is degraded to 50% or less, it was determined that reaction hindrance occurs.

As a result, immediately after the tube is produced, reaction hindrance does not occur regardless of an addition amount, and when the addition amount is 1%, reaction hindrance does not occur even after 4 months. Meanwhile, when the addition amount is 5%, reaction hindrance occurred by an experiment after 4 months, when the addition amount is 10%, reaction hindrance occurred by an experiment after 3 months, and when the addition amount is 20%, reaction hindrance occurred by an experiment after 1 month. In this way, the additive of the second liquid inactivates the freeze-dried nucleic acid amplification reagent.

### Experimental Example 6

### Influence of Additive on Elution Solution

As in Experimental Example 2, a nucleic acid amplification reagent was freeze-dried in an polypropylene tube for an elevation type PCR device. 1.4 µL of pure water was dripped into a second liquid added with an additive SS4230 of 60%, and the solution was preserved at 4°C. After 12 months, 0.2 µL of RNA to be a template was added to the solution, and the solution was dripped into a tube in which a freeze-dried nucleic acid amplification reagent was put. Then, PCR was performed by the elevation type PCR device described in JP-A-2009-136250 (after a process at 50°C for 60 seconds and at 98°C for 10 seconds, and 50 cycles of at 98°C for 5 seconds - at 60°C for 20 seconds). An additive SS4230 of 5% is added to oil in the PCR tube.

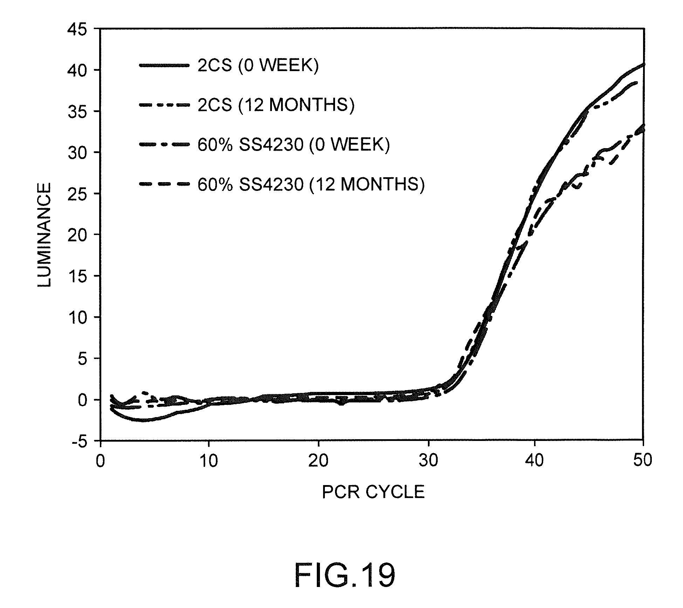

As a result, as shown in Fig. 19, even if PCR was performed using water which was brought into contact with the second liquid added with the additive SS4230 of 60% over a long period of time, reaction hindrance was not observed and a reaction normally proceeded. In this way, the additive of the second liquid has not influence on water in contact therewith. Therefore, even if an additive of a high concentration is contained in an elution solution, it is considered that there is no influence on PCR as long as PCR is performed after dilution.

## Claims

1. A cartridge for a nucleic acid amplification reaction comprising:
a tube which has a longitudinal direction and has, in the inside thereof, an elution solution plug formed of an elution solution, which undergoes phase separation from oil and causes nucleic acid to be eluted from particulates bound to the nucleic acid, and a plug of a first liquid formed of oil and a first additive; and
a nucleic acid amplification reaction container which communicates with the tube,
wherein the nucleic acid amplification reaction container contains a second liquid formed of oil and a second additive,
the first additive is selected from a group consisting of carbinol-modified silicone resin, carboxyl-modified silicone resin, amino-modified silicone resin, polyether-modified silicone resin, silanol-modified silicone resin, and fluoro-modified silicone resin,
the second additive is selected from a group consisting of carbinol-modified silicone resin, carboxyl-modified silicone resin, amino-modified silicone resin, polyether-modified silicone resin, silanol-modified silicone resin, and fluoro-modified silicone resin, and
the concentration of the first additive contained in the first liquid is higher than the concentration of the second additive contained in the second liquid.

2. The cartridge for a nucleic acid amplification reaction according to claim 1,
wherein the additive is one of X-22-160AS, X-22-3701E, KF-857, KF-859, KF-862, KF-867, KF-6017, KF-8005 (manufactured by Shin-Etsu Silicone Co., Ltd.), SR1000, SS4230, SS4267, YR3370, XS66-C1191, TSF4703, TSF4708, XF42-C5196, and XF42-C5197 (manufactured by Momentive Co., Ltd.).

3. The cartridge for a nucleic acid amplification reaction according to claim 1 or 2,
wherein the first additive and the second additive are the same silicone resin.

4. The cartridge for a nucleic acid amplification reaction according to any one of the preceding claims,
wherein the ratio of the volume, which is obtained by adding the volume of the second additive to the volume of the first additive, to the volume, which is obtained by adding the volume of the second liquid to the volume of the first liquid in the nucleic acid amplification reaction container when the plug of the first liquid is ejected to the nucleic acid amplification reaction container, is equal to or greater than 1% (v/v) and equal to or less than 20% (v/v).

5. The cartridge for a nucleic acid amplification reaction according to any one of the preceding claims,
wherein the nucleic acid amplification reaction container contains a nucleic acid amplification reaction reagent.

6. The cartridge for a nucleic acid amplification reaction according to claim 5,
wherein the nucleic acid amplification reaction reagent is held in the second liquid.

7. The cartridge for a nucleic acid amplification reaction according to claim 5 or 6,
wherein the nucleic acid amplification reaction reagent is freeze-dried.

8. The cartridge for a nucleic acid amplification reaction according to any one of claims 5 to 7,
wherein the nucleic acid amplification reaction reagent contains a surfactant.

9. The cartridge for a nucleic acid amplification reaction according to claim 8,
wherein the surfactant is NP40, Triton-X100, or Tween20.

10. The cartridge for a nucleic acid amplification reaction according to any one of the preceding claims,
wherein the container is made of polypropylene.

11. A nucleic acid amplification device on which the cartridge for a nucleic acid amplification reaction according to any one of the preceding claims is mounted.


<110> Seiko Epson Corporation

<120> CARTRIDGE FOR NUCLEIC ACID AMPLIFICATION REACTION AND NUCLEIC ACID
AMPLIFICATION DEVICE

<130> J017847801

<150> JP2014-068233
<151> 2014-03-28

<160> 3

<170> PatentIn version 3.5

<210> 1
<211> 22
<212> DNA
<213> Artificial Sequence

<220>
<223> primer

<400> 1
gaccaatcct gtcacctctg ac                                                22

<210> 2
<211> 24
<212> DNA
<213> Artificial Sequence

<220>
<223> primer

<400> 2
agggcatttt ggacaaagcg tcta                                              24

<210> 3
<211> 24
<212> DNA
<213> Artificial Sequence

<220>
<223> probe

<400> 3
tgcagtcctc gctcactggg cacg                                              24